# EUROPEAN PATENT APPLICATION

(11) **EP 3 950 005 A1**
(43) Date of publication of application: **09.02.2022**
(21) Application number: 20778368.9
(22) Date of filing: 26.03.2020
(51) Int. Cl.: A61K 48/00, A61P 35/00, A61P 35/02, A61P 43/00, C12N 5/09, C12N 15/113, A61K 31/7105, A61K 31/713

(54) **RNAI MOLECULE**

(30) Priority: 28.03.2019 JP 2019064443
(71) Applicant: NITTO DENKO CORPORATION, Ibaraki-shi Osaka 567-8680 (JP)
(72) Inventor: TANAKA Hiroyuki, Ibaraki-shi, Osaka 567-8680 (JP); TAKAHASHI Hirokazu, Ibaraki-shi, Osaka 567-8680 (JP); TAKAGI Yoshikazu, Ibaraki-shi, Osaka 567-8680 (JP); MINOMI Kenjiro, Ibaraki-shi, Osaka 567-8680 (JP)
(74) Representative: Hoffmann Eitle
(86) International application number: PCT/JP2020/013623
(87) International publication number: WO 2020/196736

(57) **Abstract**

An RNAi molecule comprising the nucleotide sequence of SEQ ID NO: 1 in an antisense strand; and a method for treating a disease, comprising administering an effective amount of the RNAi molecule to a subject in need thereof.

SEQUENCE LISTING

<110>  Nitto Denko Corporation

<120>  RNAi molecule

<130>  G2298WO

<150>  JP 2019-064443
<151>  2019-03-28

<160>  49

<170>  PatentIn version 3.5

<210>  1
<211>  7
<212>  DNA
<213>  Artificial sequence

<220>
<223>  Basic sequence

<220>
<221>  misc_feature
<223>  Combined DNA/RNA molecule

<220>
<221>  misc_feature
<222>  (1)..(7)
<223>  one of the residues is DNA and the remaining residues are RNA

<220>
<221>  misc_feature
<222>  (2)..(2)
<223>  n stands for u or t

<220>
<221>  misc_feature
<222>  (6)..(6)
<223>  n stands for u or t

<400>  1
cngacnc                                                                 7

<210>  2
<211>  7
<212>  DNA
<213>  Artificial sequence

<220>
<223>  Basic sequence 1

<220>
<221>  misc_feature
<223>  Combined DNA/RNA molecule

<220>
<221>  misc_feature
<222>  (1)..(1)

<223>  DNA

<220>
<221>  misc_feature
<222>  (2)..(7)
<223>  RNA

<400>  2
cugacuc                                                                 7


<210>  3
<211>  7
<212>  DNA
<213>  Artificial sequence

<220>
<223>  Basic sequence 2


<220>
<221>  misc_feature
<223>  Combined DNA/RNA molecule

<220>
<221>  misc_feature
<222>  (1)..(1)
<223>  RNA

<220>
<221>  misc_feature
<222>  (2)..(2)
<223>  DNA

<220>
<221>  misc_feature
<222>  (3)..(7)
<223>  RNA

<400>  3
ctgacuc                                                                 7


<210>  4
<211>  7
<212>  DNA
<213>  Artificial sequence

<220>
<223>  Basic sequence 3


<220>
<221>  misc_feature
<223>  Combined DNA/RNA molecule

<220>
<221>  misc_feature
<222>  (1)..(2)
<223>  RNA

<220>
<221>  misc_feature

<222>  (3)..(3)
<223>  DNA

<220>
<221>  misc_feature
<222>  (4)..(7)
<223>  RNA

<400>  4
cugacuc                                                                 7


<210>  5
<211>  7
<212>  DNA
<213>  Artificial sequence

<220>
<223>  Basic sequence 4


<220>
<221>  misc_feature
<223>  Combined DNA/RNA molecule

<220>
<221>  misc_feature
<222>  (1)..(3)
<223>  RNA

<220>
<221>  misc_feature
<222>  (4)..(4)
<223>  DNA

<220>
<221>  misc_feature
<222>  (5)..(7)
<223>  RNA

<400>  5
cugacuc                                                                 7


<210>  6
<211>  7
<212>  DNA
<213>  Artificial sequence

<220>
<223>  Basic sequence 5


<220>
<221>  misc_feature
<223>  Combined DNA/RNA molecule

<220>
<221>  misc_feature
<222>  (1)..(4)
<223>  RNA

<220>

<221>  misc_feature
<222>  (5)..(5)
<223>  DNA

<220>
<221>  misc_feature
<222>  (6)..(7)
<223>  RNA

<400>  6
cugacuc                                                                 7


<210>  7
<211>  7
<212>  DNA
<213>  Artificial sequence

<220>
<223>  Basic sequence 6


<220>
<221>  misc_feature
<223>  Combined DNA/RNA molecule

<220>
<221>  misc_feature
<222>  (1)..(5)
<223>  RNA

<220>
<221>  misc_feature
<222>  (6)..(6)
<223>  DNA

<220>
<221>  misc_feature
<222>  (7)..(7)
<223>  RNA

<400>  7
cugactc                                                                 7


<210>  8
<211>  7
<212>  DNA
<213>  Artificial sequence

<220>
<223>  Basic sequence 7


<220>
<221>  misc_feature
<223>  Combined DNA/RNA molecule

<220>
<221>  misc_feature
<222>  (1)..(6)
<223>  RNA

<220>
<221>  misc_feature
<222>  (7)..(7)
<223>  DNA

<400>  8
cugacuc                                                                 7


<210>  9
<211>  2574
<212>  DNA
<213>  Homo sapiens

<400>  9
ctcgatccgg gcgatggagg aggaagcaag cgagggggct ggttcctgag cttcgcaatt      60

cctgtgtcgc cttctgggct cccagcctgc cgggtcgcat gatccctccg gccggagctg     120

gtttttttgc cagccaccgc gaggccggct gagttaccgg catccccgca gccacctcct     180

ctcccgacct gtgatacaaa agatcttccg ggggctgcac ctgcctgcct ttgcctaagg     240

cggatttgaa tctctttctc tcccttcaga atcttatctt ggctttggat cttagaagag     300

aatcactaac cagagacgag actcagtgag tgagcaggtg ttttggacaa tggactggtt     360

gagcccatcc ctattataaa aatgtctcag agcaaccggg agctggtggt tgactttctc     420

tcctacaagc tttcccagaa aggatacagc tggagtcagt ttagtgatgt ggaagagaac     480

aggactgagg ccccagaagg gactgaatcg gagatggaga cccccagtgc catcaatggc     540

aacccatcct ggcacctggc agacagcccc gcggtgaatg gagccactgg ccacagcagc     600

agtttggatg cccgggaggt gatccccatg gcagcagtaa agcaagcgct gagggaggca     660

ggcgacgagt ttgaactgcg gtaccggcgg gcattcagtg acctgacatc ccagctccac     720

atcaccccag ggacagcata tcagagcttt gaacaggtag tgaatgaact cttccgggat     780

ggggtaaact ggggtcgcat tgtggccttt ttctccttcg gcggggcact gtgcgtggaa     840

agcgtagaca aggagatgca ggtattggtg agtcggatcg cagcttggat ggccacttac     900

ctgaatgacc acctagagcc ttggatccag gagaacggcg gctgggatac ttttgtggaa     960

ctctatggga acaatgcagc agccgagagc cgaaagggcc aggaacgctt caaccgctgg    1020

ttcctgacgg gcatgactgt ggccggcgtg gttctgctgg gctcactctt cagtcggaaa    1080

tgaccagaca ctgaccatcc actctaccct cccaccccct tctctgctcc accacatcct    1140

ccgtccagcc gccattgcca ccaggagaac cactacatgc agcccatgcc cacctgccca    1200

tcacagggtt gggcccagat ctggtccctt gcagctagtt ttctagaatt tatcacactt    1260

ctgtgagacc cccacacctc agttcccttg gcctcagaat tcacaaaatt tccacaaaat    1320

ctgtccaaag gaggctggca ggtatggaag ggtttgtggc tgggggcagg agggccctac    1380

ctgattggtg caacccttac cccttagcct ccctgaaaat gtttttctgc cagggagctt    1440

gaaagttttc agaacctctt ccccagaaag gagactagat tgcctttgtt ttgatgtttg 1500

tggcctcaga attgatcatt ttccccccac tctccccaca ctaacctggg ttccctttcc 1560

ttccatccct accccctaag agccatttag gggccacttt tgactaggga ttcaggctgc 1620

ttgggataaa gatgcaagga ccaggactcc ctcctcacct ctggactggc tagagtcctc 1680

actcccagtc caaatgtcct ccagaagcct ctggctagag gccagcccca cccaggaggg 1740

agggggctat agctacagga agcaccccat gccaaagcta gggtggccct tgcagttcag 1800

caccacccta gtcccttccc ctccctggct cccatgacca tactgaggga ccaactgggc 1860

ccaagacaga tgccccagag ctgtttatgg cctcagctgc ctcacttcct acaagagcag 1920

cctgtggcat ctttgccttg ggctgctcct catggtgggt tcaggggact cagccctgag 1980

gtgaaaggga gctatcagga acagctatgg gagccccagg gtcttcccta cctcaggcag 2040

gaagggcagg aaggagagcc tgctgcatgg ggtggggtag ggctgactag aagggccagt 2100

cctgcctggc caggcagatc tgtgccccat gcctgtccag cctgggcagc caggctgcca 2160

aggccagagt ggcctggcca ggagctcttc aggcctccct ctctcttctg ctccaccctt 2220

ggcctgtctc atccccaggg gtcccagcca ccccgggctc tctgctgtac atatttgaga 2280

ctagttttta ttccttgtga agatgatata ctatttttgt taagcgtgtc tgtatttatg 2340

tgtgaggagc tgctggcttg cagtgcgcgt gcacgtggag agctggtgcc cggagattgg 2400

acggcctgat gctccctccc ctgccctggt ccagggaagc tggccgaggg tcctggctcc 2460

tgaggggcat ctgcccctcc cccaaccccc accccacact tgttccagct ctttgaaata 2520

gtctgtgtga aggtgaaagt gcagttcagt aataaactgt gtttactcag tgaa 2574

<210> 10
<211> 2798
<212> DNA
<213> Homo sapiens

<400> 10
gaaaccttga accccattga gaagtccctt tagggtttcg gacgcctcca cctcaccctg 60

ggctggtgct taaatagaaa aaagaaaaac aaaaaccaac taaatccata ccagccacct 120

ccgggagagt actcctggct cccagtagga ggcggagagc caaggggcgt gcaagagaga 180

gggggctggg ctcccgggtg gcaggaggcc gcggctgcgg agcggccgcc ctcgatccgg 240

gcgatggagg aggaagcaag cgagggggct ggttcctgag cttcgcaatt cctgtgtcgc 300

cttctgggct cccagcctgc cgggtcgcat gatccctccg gccggagctg gtttttttgc 360

cagccaccgc gaggccggct gagttaccgg catccccgca gccacctcct ctcccgacct 420

gtgatacaaa agatcttccg ggggctgcac ctgcctgcct ttgcctaagg cggatttgaa 480

taatcttatc ttggctttgg atcttagaag agaatcacta accagagacg agactcagtg 540

agtgagcagg tgttttggac aatggactgg ttgagcccat ccctattata aaaatgtctc 600

agagcaaccg ggagctggtg gttgactttc tctcctacaa gctttcccag aaaggataca 660

gctggagtca gtttagtgat gtggaagaga acaggactga ggccccagaa gggactgaat 720

cggagatgga gacccccagt gccatcaatg gcaacccatc ctggcacctg gcagacagcc 780

ccgcggtgaa tggagccact ggccacagca gcagtttgga tgcccgggag gtgatcccca 840

tggcagcagt aaagcaagcg ctgagggagg caggcgacga gtttgaactg cggtaccggc 900

gggcattcag tgacctgaca tcccagctcc acatcacccc agggacagca tatcagagct 960

ttgaacaggt agtgaatgaa ctcttccggg atggggtaaa ctggggtcgc attgtggcct 1020

ttttctcctt cggcggggca ctgtgcgtgg aaagcgtaga caaggagatg caggtattgg 1080

tgagtcggat cgcagcttgg atggccactt acctgaatga ccacctagag ccttggatcc 1140

aggagaacgg cggctgggat acttttgtgg aactctatgg gaacaatgca gcagccgaga 1200

gccgaaaggg ccaggaacgc ttcaaccgct ggttcctgac gggcatgact gtggccggcg 1260

tggttctgct gggctcactc ttcagtcgga aatgaccaga cactgaccat ccactctacc 1320

ctcccacccc cttctctgct ccaccacatc ctccgtccag ccgccattgc caccaggaga 1380

accactacat gcagcccatg cccacctgcc catcacaggg ttgggcccag atctggtccc 1440

ttgcagctag ttttctagaa tttatcacac ttctgtgaga cccccacacc tcagttccct 1500

tggcctcaga attcacaaaa tttccacaaa atctgtccaa aggaggctgg caggtatgga 1560

agggtttgtg gctgggggca ggagggccct acctgattgg tgcaaccctt accccttagc 1620

ctccctgaaa atgtttttct gccagggagc ttgaaagttt tcagaacctc ttccccagaa 1680

aggagactag attgcctttg ttttgatgtt tgtggcctca gaattgatca ttttcccccc 1740

actctcccca cactaacctg ggttcccttt ccttccatcc ctacccccta agagccattt 1800

aggggccact tttgactagg gattcaggct gcttgggata aagatgcaag gaccaggact 1860

ccctcctcac ctctggactg gctagagtcc tcactcccag tccaaatgtc ctccagaagc 1920

ctctggctag aggccagccc cacccaggag ggagggggct atagctacag gaagcacccc 1980

atgccaaagc tagggtggcc cttgcagttc agcaccaccc tagtcccttc ccctccctgg 2040

ctcccatgac catactgagg gaccaactgg gcccaagaca gatgccccag agctgtttat 2100

ggcctcagct gcctcacttc ctacaagagc agcctgtggc atctttgcct tgggctgctc 2160

ctcatggtgg gttcagggga ctcagccctg aggtgaaagg gagctatcag gaacagctat 2220

gggagcccca gggtcttccc tacctcaggc aggaagggca ggaaggagag cctgctgcat 2280

ggggtggggt agggctgact agaagggcca gtcctgcctg gccaggcaga tctgtgcccc 2340

atgcctgtcc agcctgggca gccaggctgc caaggccaga gtggcctggc caggagctct 2400

tcaggcctcc ctctctcttc tgctccaccc ttggcctgtc tcatccccag gggtcccagc 2460

caccccgggc tctctgctgt acatatttga gactagtttt tattccttgt gaagatgata 2520

tactattttt gttaagcgtg tctgtattta tgtgtgagga gctgctggct tgcagtgcgc 2580

gtgcacgtgg agagctggtg cccggagatt ggacggcctg atgctccctc ccctgccctg 2640

gtccagggaa gctggccgag ggtcctggct cctgaggggc atctgcccct cccccaaccc 2700

ccaccccaca cttgttccag ctctttgaaa tagtctgtgt gaaggtgaaa gtgcagttca 2760

gtaataaact gtgtttactc agtgaacaaa gaaaaaaa 2798

<210> 11
<211> 2453
<212> DNA
<213> Homo sapiens

<400> 11
   cgcgcactcc ctttgcgtct cgggctcgcg cgcgttgccg cggcaccgga agtgactgag 60
   cttgcaagtt cccctgtctc ttcaggggaa actgaggccg gcttgttcgg gagagacggc 120
   gcgagcagtc agccagaatc ttatcttggc tttggatctt agaagagaat cactaaccag 180
   agacgagact cagtgagtga gcaggtgttt tggacaatgg actggttgag cccatcccta 240
   ttataaaaat gtctcagagc aaccgggagc tggtggttga ctttctctcc tacaagcttt 300
   cccagaaagg atacagctgg agtcagttta gtgatgtgga agagaacagg actgaggccc 360
   cagaagggac tgaatcggag atggagaccc ccagtgccat caatggcaac ccatcctggc 420
   acctggcaga cagccccgcg gtgaatggag ccactggcca cagcagcagt ttggatgccc 480
   gggaggtgat ccccatggca gcagtaaagc aagcgctgag ggaggcaggc gacgagtttg 540
   aactgcggta ccggcgggca ttcagtgacc tgacatccca gctccacatc accccaggga 600
   cagcatatca gagctttgaa caggtagtga atgaactctt ccgggatggg gtaaactggg 660
   gtcgcattgt ggcctttttc tccttcggcg gggcactgtg cgtggaaagc gtagacaagg 720
   agatgcaggt attggtgagt cggatcgcag cttggatggc cacttacctg aatgaccacc 780
   tagagccttg gatccaggag aacggcggct gggatacttt tgtggaactc tatgggaaca 840
   atgcagcagc cgagagccga aagggccagg aacgcttcaa ccgctggttc ctgacgggca 900
   tgactgtggc cggcgtggtt ctgctgggct cactcttcag tcggaaatga ccagacactg 960
   accatccact ctaccctccc acccccttct ctgctccacc acatcctccg tccagccgcc 1020
   attgccacca ggagaaccac tacatgcagc ccatgcccac ctgcccatca cagggttggg 1080
   cccagatctg gtcccttgca gctagttttc tagaatttat cacacttctg tgagaccccc 1140
   acacctcagt tcccttggcc tcagaattca caaaatttcc acaaaatctg tccaaaggag 1200
   gctggcaggt atggaagggt ttgtggctgg gggcaggagg gccctacctg attggtgcaa 1260
   cccttacccc ttagcctccc tgaaaatgtt tttctgccag ggagcttgaa agttttcaga 1320
   acctcttccc cagaaaggag actagattgc ctttgttttg atgtttgtgg cctcagaatt 1380
   gatcattttc cccccactct ccccacacta acctgggttc cctttccttc catccctacc 1440
   ccctaagagc catttagggg ccacttttga ctagggattc aggctgcttg ggataaagat 1500
   gcaaggacca ggactccctc ctcacctctg gactggctag agtcctcact cccagtccaa 1560
   atgtcctcca gaagcctctg gctagaggcc agccccaccc aggagggagg gggctatagc 1620
   tacaggaagc accccatgcc aaagctaggg tggcccttgc agttcagcac caccctagtc 1680
   ccttcccctc cctggctccc atgaccatac tgagggacca actgggccca agacagatgc 1740
   cccagagctg tttatggcct cagctgcctc acttcctaca agagcagcct gtggcatctt 1800
   tgccttgggc tgctcctcat ggtgggttca ggggactcag ccctgaggtg aaagggagct 1860
   atcaggaaca gctatgggag ccccagggtc ttccctacct caggcaggaa gggcaggaag 1920
   gagagcctgc tgcatggggt ggggtagggc tgactagaag ggccagtcct gcctggccag 1980
   gcagatctgt gccccatgcc tgtccagcct gggcagccag gctgccaagg ccagagtggc 2040
   ctggccagga gctcttcagg cctccctctc tcttctgctc cacccttggc ctgtctcatc 2100
   cccaggggtc ccagccaccc cgggctctct gctgtacata tttgagacta gtttttattc 2160
   cttgtgaaga tgatatacta tttttgttaa gcgtgtctgt atttatgtgt gaggagctgc 2220
   tggcttgcag tgcgcgtgca cgtggagagc tggtgcccgg agattggacg gcctgatgct 2280
   ccctcccctg ccctggtcca gggaagctgg ccgagggtcc tggctcctga ggggcatctg 2340
   cccctccccc aacccccacc ccacacttgt tccagctctt tgaaatagtc tgtgtgaagg 2400
   tgaaagtgca gttcagtaat aaactgtgtt tactcagtga acaaagaaaa aaa 2453

<210> 12
<211> 2582
<212> DNA
<213> Homo sapiens

<400> 12
   cgcgcactcc ctttgcgtct cgggctcgcg cgcgttgccg cggcaccgga agtgactgag 60
   cttgcaagtt cccctgtctc ttcaggggaa actgaggccg gcttgttcgg gagagacggc 120
   gcgagcagtc agccaggtag gccggcagcc aggtaggccg gcccgggtcc gcggcgcgga 180
   actcggccgc gaagagctct tgcgtctgga agctaccggg ccgatgaagg gggatgtggc 240
   cccccacggc tcgcggggct cgcagaatct tatcttggct ttggatctta gaagagaatc 300
   actaaccaga gacgagactc agtgagtgag caggtgtttt ggacaatgga ctggttgagc 360
   ccatccctat tataaaaatg tctcagagca accgggagct ggtggttgac tttctctcct 420
   acaagctttc ccagaaagga tacagctgga gtcagtttag tgatgtggaa gagaacagga 480
   ctgaggcccc agaagggact gaatcggaga tggagacccc cagtgccatc aatggcaacc 540
   catcctggca cctggcagac agccccgcgg tgaatggagc cactggccac agcagcagtt 600
   tggatgcccg ggaggtgatc cccatggcag cagtaaagca agcgctgagg gaggcaggcg 660
   acgagtttga actgcggtac cggcgggcat tcagtgacct gacatcccag ctccacatca 720
   ccccagggac agcatatcag agctttgaac aggtagtgaa tgaactcttc cgggatgggg 780
   taaactgggg tcgcattgtg gcctttttct ccttcggcgg ggcactgtgc gtggaaagcg 840
   tagacaagga gatgcaggta ttggtgagtc ggatcgcagc ttggatggcc acttacctga 900
   atgaccacct agagccttgg atccaggaga acggcggctg ggatactttt gtggaactct 960
   atgggaacaa tgcagcagcc gagagccgaa agggccagga acgcttcaac cgctggttcc 1020
   tgacgggcat gactgtggcc ggcgtggttc tgctgggctc actcttcagt cggaaatgac 1080
   cagacactga ccatccactc taccctccca cccccttctc tgctccacca catcctccgt 1140
   ccagccgcca ttgccaccag gagaaccact acatgcagcc catgcccacc tgcccatcac 1200
   agggttgggc ccagatctgg tcccttgcag ctagttttct agaatttatc acacttctgt 1260
   gagaccccca cacctcagtt cccttggcct cagaattcac aaaatttcca caaaatctgt 1320
   ccaaaggagg ctggcaggta tggaagggtt tgtggctggg ggcaggaggg ccctacctga 1380
   ttggtgcaac ccttacccct tagcctccct gaaaatgttt ttctgccagg gagcttgaaa 1440
   gttttcagaa cctcttcccc agaaaggaga ctagattgcc tttgttttga tgtttgtggc 1500
   ctcagaattg atcattttcc ccccactctc cccacactaa cctgggttcc ctttccttcc 1560
   atccctaccc cctaagagcc atttaggggc cacttttgac tagggattca ggctgcttgg 1620
   gataaagatg caaggaccag gactccctcc tcacctctgg actggctaga gtcctcactc 1680
   ccagtccaaa tgtcctccag aagcctctgg ctagaggcca gccccaccca ggagggaggg 1740
   ggctatagct acaggaagca ccccatgcca aagctagggt ggcccttgca gttcagcacc 1800
   accctagtcc cttcccctcc ctggctccca tgaccatact gagggaccaa ctgggcccaa 1860
   gacagatgcc ccagagctgt ttatggcctc agctgcctca cttcctacaa gagcagcctg 1920
   tggcatcttt gccttgggct gctcctcatg gtgggttcag gggactcagc cctgaggtga 1980
   aagggagcta tcaggaacag ctatgggagc cccagggtct tccctacctc aggcaggaag 2040
   ggcaggaagg agagcctgct gcatggggtg gggtagggct gactagaagg gccagtcctg 2100
   cctggccagg cagatctgtg ccccatgcct gtccagcctg ggcagccagg ctgccaaggc 2160
   cagagtggcc tggccaggag ctcttcaggc ctccctctct cttctgctcc acccttggcc 2220
   tgtctcatcc ccaggggtcc cagccacccc gggctctctg ctgtacatat ttgagactag 2280
   tttttattcc ttgtgaagat gatatactat ttttgttaag cgtgtctgta tttatgtgtg 2340
   aggagctgct ggcttgcagt gcgcgtgcac gtggagagct ggtgcccgga gattggacgg 2400
   cctgatgctc cctcccctgc cctggtccag ggaagctggc cgagggtcct ggctcctgag 2460
   gggcatctgc ccctccccca acccccaccc cacacttgtt ccagctcttt gaaatagtct 2520
   gtgtgaaggt gaaagtgcag ttcagtaata aactgtgttt actcagtgaa caaagaaaaa 2580
   aa 2582

<210> 13
<211> 2471
<212> DNA
<213> Homo sapiens

<400> 13
cgcgcactcc ctttgcgtct cgggctcgcg cgcgttgccg cggcaccgga agtgactgag 60

cttgcaagtt cccctgtctc ttcaggggaa actgaggccg gcttgttcgg gagagacggc 120

gcgagcagtc agccagctct ttctctccct tcagaatctt atcttggctt tggatcttag 180

aagagaatca ctaaccagag acgagactca gtgagtgagc aggtgttttg gacaatggac 240

tggttgagcc catccctatt ataaaaatgt ctcagagcaa ccgggagctg gtggttgact 300

ttctctccta caagctttcc cagaaaggat acagctggag tcagtttagt gatgtggaag 360

agaacaggac tgaggcccca gaagggactg aatcggagat ggagaccccc agtgccatca 420

atggcaaccc atcctggcac ctggcagaca gccccgcggt gaatggagcc actggccaca 480

gcagcagttt ggatgcccgg gaggtgatcc ccatggcagc agtaaagcaa gcgctgaggg 540

aggcaggcga cgagtttgaa ctgcggtacc ggcgggcatt cagtgacctg acatcccagc 600

tccacatcac cccagggaca gcatatcaga gctttgaaca ggtagtgaat gaactcttcc 660

gggatggggt aaactggggt cgcattgtgg cctttttctc cttcggcggg gcactgtgcg 720

tggaaagcgt agacaaggag atgcaggtat tggtgagtcg gatcgcagct tggatggcca 780

cttacctgaa tgaccaccta gagccttgga tccaggagaa cggcggctgg gatacttttg 840

tggaactcta tgggaacaat gcagcagccg agagccgaaa gggccaggaa cgcttcaacc 900

gctggttcct gacgggcatg actgtggccg gcgtggttct gctgggctca ctcttcagtc 960

ggaaatgacc agacactgac catccactct accctcccac ccccttctct gctccaccac 1020

atcctccgtc cagccgccat tgccaccagg agaaccacta catgcagccc atgcccacct 1080

gcccatcaca gggttgggcc cagatctggt cccttgcagc tagttttcta gaatttatca 1140

cacttctgtg agacccccac acctcagttc ccttggcctc agaattcaca aaatttccac 1200

aaaatctgtc caaaggaggc tggcaggtat ggaagggttt gtggctgggg gcaggagggc 1260

cctacctgat tggtgcaacc cttacccctt agcctccctg aaaatgtttt tctgccaggg 1320

agcttgaaag ttttcagaac ctcttcccca gaaaggagac tagattgcct ttgttttgat 1380

gtttgtggcc tcagaattga tcattttccc cccactctcc ccacactaac ctgggttccc 1440

tttccttcca tccctacccc ctaagagcca tttaggggcc acttttgact agggattcag 1500

gctgcttggg ataaagatgc aaggaccagg actccctcct cacctctgga ctggctagag 1560

tcctcactcc cagtccaaat gtcctccaga agcctctggc tagaggccag ccccacccag 1620

gagggagggg gctatagcta caggaagcac cccatgccaa agctagggtg gcccttgcag 1680

ttcagcacca ccctagtccc ttcccctccc tggctcccat gaccatactg agggaccaac 1740

tgggcccaag acagatgccc cagagctgtt tatggcctca gctgcctcac ttcctacaag 1800

agcagcctgt ggcatctttg ccttgggctg ctcctcatgg tgggttcagg ggactcagcc 1860

ctgaggtgaa agggagctat caggaacagc tatgggagcc ccagggtctt ccctacctca 1920

ggcaggaagg gcaggaagga gagcctgctg catggggtgg ggtagggctg actagaaggg 1980

ccagtcctgc ctggccaggc agatctgtgc cccatgcctg tccagcctgg gcagccaggc 2040

tgccaaggcc agagtggcct ggccaggagc tcttcaggcc tccctctctc ttctgctcca 2100

cccttggcct gtctcatccc caggggtccc agccaccccg ggctctctgc tgtacatatt 2160

tgagactagt ttttattcct tgtgaagatg atatactatt tttgttaagc gtgtctgtat 2220

ttatgtgtga ggagctgctg gcttgcagtg cgcgtgcacg tggagagctg gtgcccggag 2280

attggacggc ctgatgctcc ctcccctgcc ctggtccagg gaagctggcc gagggtcctg 2340

gctcctgagg ggcatctgcc cctcccccaa cccccacccc acacttgttc cagctctttg 2400

aaatagtctg tgtgaaggtg aaagtgcagt tcagtaataa actgtgttta ctcagtgaac 2460

aaagaaaaaa a 2471

<210> 14
<211> 2600
<212> DNA
<213> Homo sapiens

<400> 14
   cgcgcactcc ctttgcgtct cgggctcgcg cgcgttgccg cggcaccgga agtgactgag 60
   cttgcaagtt cccctgtctc ttcaggggaa actgaggccg gcttgttcgg gagagacggc 120
   gcgagcagtc agccaggtag gccggcagcc aggtaggccg gcccgggtcc gcggcgcgga 180
   actcggccgc gaagagctct tgcgtctgga agctaccggg ccgatgaagg gggatgtggc 240
   cccccacggc tcgcggggct cgcagctctt tctctccctt cagaatctta tcttggcttt 300
   ggatcttaga agagaatcac taaccagaga cgagactcag tgagtgagca ggtgttttgg 360
   acaatggact ggttgagccc atccctatta taaaaatgtc tcagagcaac cgggagctgg 420
   tggttgactt tctctcctac aagctttccc agaaaggata cagctggagt cagtttagtg 480
   atgtggaaga gaacaggact gaggccccag aagggactga atcggagatg gagaccccca 540
   gtgccatcaa tggcaaccca tcctggcacc tggcagacag ccccgcggtg aatggagcca 600
   ctggccacag cagcagtttg gatgcccggg aggtgatccc catggcagca gtaaagcaag 660
   cgctgaggga ggcaggcgac gagtttgaac tgcggtaccg gcgggcattc agtgacctga 720
   catcccagct ccacatcacc ccagggacag catatcagag ctttgaacag gtagtgaatg 780
   aactcttccg ggatggggta aactggggtc gcattgtggc ctttttctcc ttcggcgggg 840
   cactgtgcgt ggaaagcgta gacaaggaga tgcaggtatt ggtgagtcgg atcgcagctt 900
   ggatggccac ttacctgaat gaccacctag agccttggat ccaggagaac ggcggctggg 960
   atacttttgt ggaactctat gggaacaatg cagcagccga gagccgaaag ggccaggaac 1020
   gcttcaaccg ctggttcctg acgggcatga ctgtggccgg cgtggttctg ctgggctcac 1080
   tcttcagtcg gaaatgacca gacactgacc atccactcta ccctcccacc cccttctctg 1140
   ctccaccaca tcctccgtcc agccgccatt gccaccagga gaaccactac atgcagccca 1200
   tgcccacctg cccatcacag ggttgggccc agatctggtc ccttgcagct agttttctag 1260
   aatttatcac acttctgtga gacccccaca cctcagttcc cttggcctca gaattcacaa 1320
   aatttccaca aaatctgtcc aaaggaggct ggcaggtatg gaagggtttg tggctggggg 1380
   caggagggcc ctacctgatt ggtgcaaccc ttacccctta gcctccctga aaatgttttt 1440
   ctgccaggga gcttgaaagt tttcagaacc tcttccccag aaaggagact agattgcctt 1500
   tgttttgatg tttgtggcct cagaattgat cattttcccc ccactctccc cacactaacc 1560
   tgggttccct ttccttccat ccctaccccc taagagccat ttaggggcca cttttgacta 1620
   gggattcagg ctgcttggga taaagatgca aggaccagga ctccctcctc acctctggac 1680
   tggctagagt cctcactccc agtccaaatg tcctccagaa gcctctggct agaggccagc 1740
   cccacccagg agggaggggg ctatagctac aggaagcacc ccatgccaaa gctagggtgg 1800
   cccttgcagt tcagcaccac cctagtccct tcccctccct ggctcccatg accatactga 1860
   gggaccaact gggcccaaga cagatgcccc agagctgttt atggcctcag ctgcctcact 1920
   tcctacaaga gcagcctgtg gcatctttgc cttgggctgc tcctcatggt gggttcaggg 1980
   gactcagccc tgaggtgaaa gggagctatc aggaacagct atgggagccc cagggtcttc 2040
   cctacctcag gcaggaaggg caggaaggag agcctgctgc atggggtggg gtagggctga 2100
   ctagaagggc cagtcctgcc tggccaggca gatctgtgcc ccatgcctgt ccagcctggg 2160
   cagccaggct gccaaggcca gagtggcctg gccaggagct cttcaggcct ccctctctct 2220
   tctgctccac ccttggcctg tctcatcccc aggggtccca gccaccccgg gctctctgct 2280
   gtacatattt gagactagtt tttattcctt gtgaagatga tatactattt ttgttaagcg 2340
   tgtctgtatt tatgtgtgag gagctgctgg cttgcagtgc gcgtgcacgt ggagagctgg 2400
   tgcccggaga ttggacggcc tgatgctccc tcccctgccc tggtccaggg aagctggccg 2460
   agggtcctgg ctcctgaggg gcatctgccc ctcccccaac ccccacccca cacttgttcc 2520
   agctctttga aatagtctgt gtgaaggtga aagtgcagtt cagtaataaa ctgtgtttac 2580
   tcagtgaaca aagaaaaaaa 2600

<210> 15
<211> 2453
<212> DNA
<213> Homo sapiens

<400> 15
ggcatttcgg agaagacggg ggtagaaaag gctggtggga gattcagagt ccactggtgc 60

tttcgatttg acttaagtga agtatcttgg aacctagacc cagaccttcg taagacccac 120

aaagaaacca gttctgaatc ttatcttggc tttggatctt agaagagaat cactaaccag 180

agacgagact cagtgagtga gcaggtgttt tggacaatgg actggttgag cccatcccta 240

ttataaaaat gtctcagagc aaccgggagc tggtggttga ctttctctcc tacaagcttt 300

cccagaaagg atacagctgg agtcagttta gtgatgtgga agagaacagg actgaggccc 360

cagaagggac tgaatcggag atggagaccc ccagtgccat caatggcaac ccatcctggc 420

acctggcaga cagccccgcg gtgaatggag ccactggcca cagcagcagt ttggatgccc 480

gggaggtgat ccccatggca gcagtaaagc aagcgctgag ggaggcaggc gacgagtttg 540

aactgcggta ccggcgggca ttcagtgacc tgacatccca gctccacatc accccaggga 600

cagcatatca gagctttgaa caggtagtga atgaactctt ccgggatggg gtaaactggg 660

gtcgcattgt ggcctttttc tccttcggcg gggcactgtg cgtggaaagc gtagacaagg 720

agatgcaggt attggtgagt cggatcgcag cttggatggc cacttacctg aatgaccacc 780

tagagccttg gatccaggag aacggcggct gggatacttt tgtggaactc tatgggaaca 840

atgcagcagc cgagagccga aagggccagg aacgcttcaa ccgctggttc ctgacgggca 900

tgactgtggc cggcgtggtt ctgctgggct cactcttcag tcggaaatga ccagacactg 960

accatccact ctaccctccc acccccttct ctgctccacc acatcctccg tccagccgcc 1020

attgccacca ggagaaccac tacatgcagc ccatgcccac ctgcccatca cagggttggg 1080

cccagatctg gtcccttgca gctagttttc tagaatttat cacacttctg tgagaccccc 1140

acacctcagt tcccttggcc tcagaattca caaaatttcc acaaaatctg tccaaaggag 1200

gctggcaggt atggaagggt ttgtggctgg gggcaggagg gccctacctg attggtgcaa 1260

cccttacccc ttagcctccc tgaaaatgtt tttctgccag ggagcttgaa agttttcaga 1320

acctcttccc cagaaaggag actagattgc ctttgttttg atgtttgtgg cctcagaatt 1380

gatcattttc cccccactct ccccacacta acctgggttc cctttccttc catccctacc 1440

ccctaagagc catttagggg ccacttttga ctagggattc aggctgcttg ggataaagat 1500

gcaaggacca ggactccctc ctcacctctg gactggctag agtcctcact cccagtccaa 1560

atgtcctcca gaagcctctg gctagaggcc agccccaccc aggagggagg gggctatagc      1620

tacaggaagc accccatgcc aaagctaggg tggcccttgc agttcagcac caccctagtc      1680

ccttcccctc cctggctccc atgaccatac tgagggacca actgggccca agacagatgc      1740

cccagagctg tttatggcct cagctgcctc acttcctaca agagcagcct gtggcatctt      1800

tgccttgggc tgctcctcat ggtgggttca ggggactcag ccctgaggtg aaagggagct      1860

atcaggaaca gctatgggag ccccagggtc ttccctacct caggcaggaa gggcaggaag      1920

gagagcctgc tgcatggggt ggggtagggc tgactagaag ggccagtcct gcctggccag      1980

gcagatctgt gccccatgcc tgtccagcct gggcagccag gctgccaagg ccagagtggc      2040

ctggccagga gctcttcagg cctccctctc tcttctgctc cacccttggc ctgtctcatc      2100

cccaggggtc ccagccaccc cgggctctct gctgtacata tttgagacta gtttttattc      2160

cttgtgaaga tgatatacta tttttgttaa gcgtgtctgt atttatgtgt gaggagctgc      2220

tggcttgcag tgcgcgtgca cgtggagagc tggtgcccgg agattggacg gcctgatgct      2280

ccctcccctg ccctggtcca gggaagctgg ccgagggtcc tggctcctga ggggcatctg      2340

cccctccccc aacccccacc ccacacttgt tccagctctt tgaaatagtc tgtgtgaagg      2400

tgaaagtgca gttcagtaat aaactgtgtt tactcagtga acaaagaaaa aaa            2453

<210>  16
<211>  7
<212>  DNA
<213>  Homo sapiens

<400>  16
gagtcag                                                                  7


<210>  17
<211>  15
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  17
acngacncca gcugu                                                        15


<210>  18
<211>  16
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  18
acngacncca gcugua                                                       16


<210>  19
<211>  17
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)

<223>  n stands for u or t

<220>
<221>  misc_feature
<222>  (7)..(7)
<223>  n stands for u or t

<400>  19
acngacncca gcuguau                                                    17


<210>  20
<211>  18
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  20
acngacncca gcuguauc                                                   18


<210>  21
<211>  19
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature

<222>  (3)..(3)
<223>  n stands for u or t

<220>
<221>  misc_feature
<222>  (7)..(7)
<223>  n stands for u or t

<400>  21
acngacncca gcuguaucc                                                    19


<210>  22
<211>  20
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  22
acngacncca gcuguauccu                                                   20


<210>  23
<211>  21
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>

<221>  misc_feature
<222>  (3)..(3)
<223>  n stands for u or t

<220>
<221>  misc_feature
<222>  (7)..(7)
<223>  n stands for u or t

<400>  23
acngacncca gcuguauccu u                                                21


<210>  24
<211>  22
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  24
acngacncca gcuguauccu uu                                               22


<210>  25
<211>  23
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  25
acngacncca gcuguauccu uuc                                              23


<210>  26
<211>  24
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  26
acngacncca gcuguauccu uucu                                             24


<210>  27
<211>  25
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  27
acngacncca gcuguauccu uucug                                          25


<210>  28
<211>  26
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  28
acngacncca gcuguauccu uucugg                                         26


<210>  29
<211>  27
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)

<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  29
acngacncca gcuguauccu uucuggg                                          27


<210>  30
<211>  28
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  30
acngacncca gcuguauccu uucuggga                                         28


<210>  31
<211>  29
<212>  DNA
<213>  Artificial sequence

<220>
<223>  Exemplary antisense sequence


<220>
<221>  misc_feature
<223>  Combined DNA/RNA molecule

<220>
<221>  misc_feature

<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  31
acngacncca gcuguauccu uucugggaa                                          29


<210>  32
<211>  30
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  32
acngacncca gcuguauccu uucugggaaa                                         30


<210>  33
<211>  31
<212>  DNA
<213>  Artificial sequence

<220>
<223>  Exemplary antisense sequence


<220>
<221>  misc_feature
<223>  Combined DNA/RNA molecule

<220>

<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  33
acngacncca gcuguauccu uucugggaaa g                                    31


<210>  34
<211>  32
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  34
acngacncca gcuguauccu uucugggaaa gc                                   32


<210>  35
<211>  33
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  35
acngacncca gcuguauccu uucugggaaa gcu                                    33


<210>  36
<211>  34
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  36
acngacncca gcuguauccu uucugggaaa gcuu                                   34


<210>  37
<211>  35
<212>  DNA
<213>  Artificial sequence

<220>
<223>  Exemplary antisense sequence


<220>
<221>  misc_feature
<223>  Combined DNA/RNA molecule

<220>
<221>  misc_feature
<222>  (2)..(8)
<223>  one of the residues is DNA and the remaining residues are RNA

<220>
<221>  misc_feature
<222>  (3)..(3)
<223>  n stands for u or t

<220>
<221>  misc_feature
<222>  (7)..(7)
<223>  n stands for u or t

<400>  37
acngacncca gcuguauccu uucugggaaa gcuug                                35


<210>  38
<211>  21
<212>  DNA
<213>  Artificial sequence

<220>
<223>  Sense strand of Compounds X and D1-D8


<220>
<221>  misc_feature
<223>  Combined DNA/RNA molecule

<220>
<221>  misc_feature
<222>  (1)..(19)
<223>  RNA

<400>  38
ggauacagcu ggagucagut t                                               21


<210>  39
<211>  21
<212>  DNA
<213>  Artificial sequence

<220>
<223>  Antisense strand of Compound X


<220>
<221>  misc_feature
<223>  Combined DNA/RNA molecule

<220>
<221>  misc_feature
<222>  (1)..(19)
<223>  RNA

<400>  39
acugacucca gcuguaucct t                                               21

<210>  40
<211>  21
<212>  DNA
<213>  Artificial sequence

<220>
<223>  Sense strand of Compound Y


<220>
<221>  misc_feature
<223>  Combined DNA/RNA molecule

<220>
<221>  misc_feature
<222>  (1)..(19)
<223>  RNA

<400>  40
gguauuggug agucggauct t                                                21


<210>  41
<211>  21
<212>  DNA
<213>  Artificial sequence

<220>
<223>  Antisense strand of Compound Y


<220>
<221>  misc_feature
<223>  Combined DNA/RNA molecule

<220>
<221>  misc_feature
<222>  (1)..(19)
<223>  RNA

<400>  41
gauccgacuc accaauacct t                                                21


<210>  42
<211>  21
<212>  DNA
<213>  Artificial sequence

<220>
<223>  Antisense strand of Compound D1


<220>
<221>  misc_feature
<223>  Combined DNA/RNA molecule

<220>
<221>  misc_feature
<222>  (1)..(1)
<223>  RNA

<220>
<221>  misc_feature
<222>  (2)..(2)
<223>  DNA

<220>
<221>  misc_feature
<222>  (3)..(19)
<223>  RNA

<400>  42
acugacucca gcuguaucct t                                                21


<210>  43
<211>  21
<212>  DNA
<213>  Artificial sequence

<220>
<223>  Antisense strand of Compound D2


<220>
<221>  misc_feature
<223>  Combined DNA/RNA molecule

<220>
<221>  misc_feature
<222>  (1)..(2)
<223>  RNA

<220>
<221>  misc_feature
<222>  (4)..(19)
<223>  RNA

<400>  43
actgacucca gcuguaucct t                                                21


<210>  44
<211>  21
<212>  DNA
<213>  Artificial sequence

<220>
<223>  Antisense strand of Compound D3


<220>
<221>  misc_feature
<223>  Combined DNA/RNA molecule

<220>
<221>  misc_feature
<222>  (1)..(3)
<223>  RNA

<220>
<221>  misc_feature
<222>  (4)..(4)
<223>  DNA

<220>
<221>  misc_feature
<222>  (5)..(19)
<223>  RNA

<400>  44
acugacucca gcuguaucct t                                                21


<210>  45
<211>  21
<212>  DNA
<213>  Artificial sequence

<220>
<223>  Antisense strand of Compound D4


<220>
<221>  misc_feature
<223>  Combined DNA/RNA molecule

<220>
<221>  misc_feature
<222>  (1)..(4)
<223>  RNA

<220>
<221>  misc_feature
<222>  (5)..(5)
<223>  DNA

<220>
<221>  misc_feature
<222>  (6)..(19)
<223>  RNA

<400>  45
acugacucca gcuguaucct t                                                21


<210>  46
<211>  21
<212>  DNA
<213>  Artificial sequence

<220>
<223>  Antisense strand of Compound D5


<220>
<221>  misc_feature
<223>  Combined DNA/RNA molecule

<220>
<221>  misc_feature
<222>  (1)..(5)
<223>  RNA

<220>
<221>  misc_feature
<222>  (6)..(6)

<223>  DNA

<220>
<221>  misc_feature
<222>  (7)..(19)
<223>  RNA

<400>  46
acugacucca gcuguaucct t                                                21


<210>  47
<211>  21
<212>  DNA
<213>  Artificial sequence

<220>
<223>  Antisense strand of Compound D6


<220>
<221>  misc_feature
<223>  Combined DNA/RNA molecule

<220>
<221>  misc_feature
<222>  (1)..(6)
<223>  RNA

<220>
<221>  misc_feature
<222>  (8)..(19)
<223>  RNA

<400>  47
acugactcca gcuguaucct t                                                21


<210>  48
<211>  21
<212>  DNA
<213>  Artificial sequence

<220>
<223>  Antisense strand of Compound D7


<220>
<221>  misc_feature
<223>  Combined DNA/RNA molecule

<220>
<221>  misc_feature
<222>  (1)..(7)
<223>  RNA

<220>
<221>  misc_feature
<222>  (8)..(8)
<223>  DNA

<220>
<221>  misc_feature

<222>  (9)..(19)
<223>  RNA

<400>  48
acugacucca gcuguaucct t                                                21


<210>  49
<211>  21
<212>  DNA
<213>  Artificial sequence

<220>
<223>  Antisense strand of Compound D8


<220>
<221>  misc_feature
<223>  Combined DNA/RNA molecule

<220>
<221>  misc_feature
<222>  (1)..(3)
<223>  RNA

<220>
<221>  misc_feature
<222>  (4)..(4)
<223>  DNA

<220>
<221>  misc_feature
<222>  (5)..(5)
<223>  RNA

<220>
<221>  misc_feature
<222>  (6)..(6)
<223>  DNA

<220>
<221>  misc_feature
<222>  (8)..(8)
<223>  DNA

<220>
<221>  misc_feature
<222>  (9)..(19)
<223>  RNA

<400>  49
acugacucca gcuguaucct t                                                21

## Description

### TECHNICAL FIELD

The present disclosure relates to an RNAi molecule, a method for treating a disease using the RNAi molecule, etc.

### BACKGROUND ART

Cancer chemotherapy started with use of drugs, such as alkylating agents, which exhibit cytotoxicity in a cell-non-specific manner. In recent years, molecular targeted drugs have drawn interest which target molecules that are unique to cancers and have smaller influence on cells other than cancer cells. In these circumstances, it has been reported that BcL-XL is upregulated in some types of cancers (Non-Patent Literature 1).

### CITATION LIST

### Non-Patent Literature

Non-Patent Literature 1: Sarosiek and Leta, FEBS J. 2016;283(19):3523-3533

### DISCLOSURE OF THE INVENTION

### PROBLEMS TO BE SOLVED BY THE INVENTION

Although the development of cell proliferation suppressors is underway worldwide, there is still a demand for drugs having high efficacy.

### SOLUTION TO PROBLEM

Some aspects of the present disclosure relate to the following.
[1] An RNAi molecule comprising the nucleotide sequence of SEQ ID NO: 1 in an antisense strand.
[2] The RNAi molecule according to [1], wherein the nucleotide sequence of SEQ ID NO: 1 is located at positions 2 to 8 from 5' of the antisense strand.
[3] The RNAi molecule according to [1] or [2], which suppresses the protein expression of a member of the BcL2 family.
[4] The RNAi molecule according to [3], wherein the member of the BcL2 family is BcL-XL.
[5] The RNAi molecule according to any one of [1] to [4], wherein the antisense strand comprises the nucleotide sequence of SEQ ID NO: 21.
[6] The RNAi molecule according to any one of [1] to [5], wherein the nucleotide sequence of SEQ ID NO: 1 is selected from the nucleotide sequence of SEQ ID NOs: 5 to 7.
[7] A pharmaceutical composition comprising the RNAi molecule according to any one of [1] to [6], and optionally a pharmaceutically acceptable additive.
[8] The RNAi molecule according to any one of [1] to [6] or the composition according to [7] for use in a treatment of a cancer.
[9] The RNAi molecule or composition according to [8], wherein the cancer expresses BcL-XL.
[10] The RNAi molecule or the pharmaceutical composition according to [9], wherein the cancer is selected from the group consisting of brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia.
[11] A method for treating a cancer, comprising administering an effective amount of the RNAi molecule according to any one of [1] to [6] and [8] to [10] or the composition according to any one of [7] to [10] to a subject in need thereof

### ADVANTAGEOUS EFFECTS OF INVENTION

The RNAi molecule according to the present disclosure is capable of exerting one or more of the following effects according to the aspects.
(1) Can suppress the proliferation of cells.
(2) Can induce the apoptosis of cells.
(3) Suppress the expression of a particular gene selected at least from BcL-2, Smad1, P21, MRS2 and RFCl in addition to the originally targeted BcL-XL.
(4) Have higher ability to suppress cell proliferation than that of other RNAi molecules targeting BcL-XL.
(5) Have higher ability to kill cells than that of other RNAi molecules targeting BcL-XL.

### BRIEF DESCRIPTION OF DRAWINGS

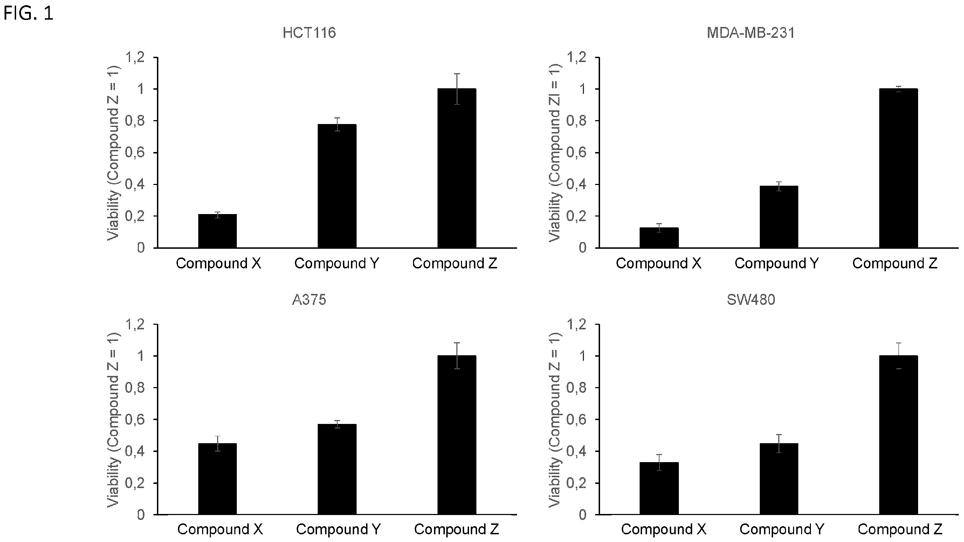

Figure 1 is a diagram showing the comparison of the ability of siRNA targeting BcL-XL to suppress the proliferation of cancer cells. The ordinate shows a relative value of a viability with a value for a control group (Compound Z) defined as 1.

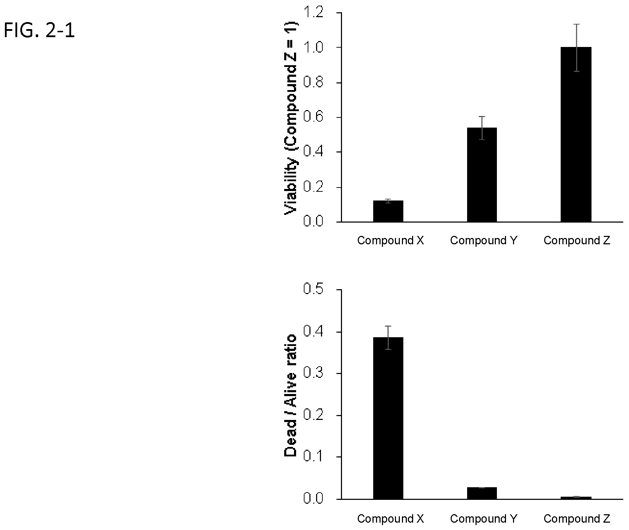

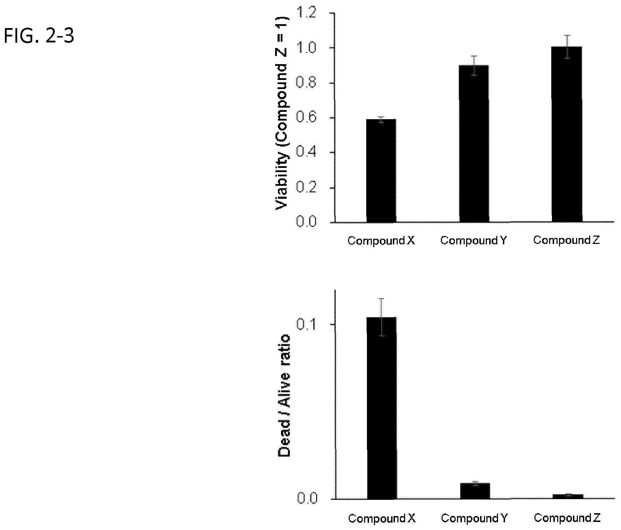

Figure 2-1 is a diagram showing the comparison of the ability of siRNA targeting BcL-XL to suppress cancer cell proliferation and the ability thereof to kill cancer cells with respect to A549 cells. The upper graph shows a relative value of a viability with a value for a control group (Compound Z) defined as 1. The lower graph shows a numerical value obtained by dividing a dead cell number by a live cell number.

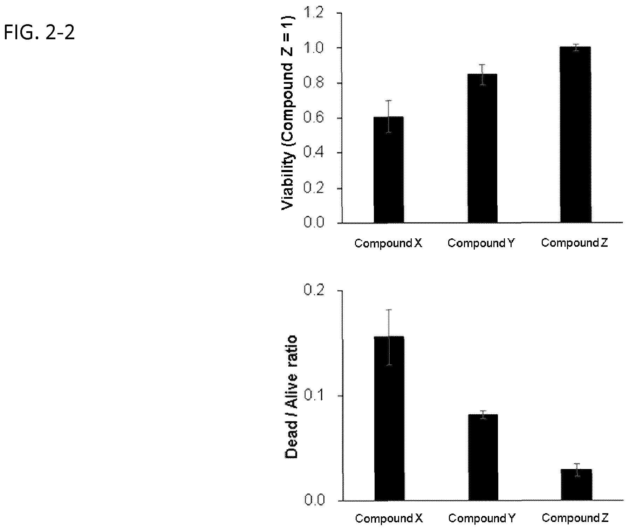

Figure 2-2 is a diagram showing the comparison of the ability of siRNA targeting BcL-XL to suppress cancer cell proliferation and the ability thereof to kill cancer cells with respect to SW1990 cells. The upper graph shows a relative value of a viability with a value for a control group (Compound Z) defined as 1. The lower graph shows a numerical value obtained by dividing a dead cell number by a live cell number.
Figure 2-3 is a diagram showing the comparison of the ability of siRNA targeting BcL-XL to suppress cancer cell proliferation and the ability thereof to kill cancer cells with respect to SUIT-2 cells. The upper graph shows a relative value of a viability with a value for a control group (Compound Z) defined as 1. The lower graph shows a numerical value obtained by dividing a dead cell number by a live cell number.
Figure 3 is a diagram showing the comparison of the ability of siRNA targeting BcL-XL to suppress the expression of other particular genes. The ordinate shows a relative value of the expression level of each gene normalized with the expression level of GAPDH with a value for a control group (Compound Z) defined as 1.
Figure 4 is a diagram showing the apoptosis-inducing activity of CUGACUC-containing siRNA.
Figure 5 is a diagram showing the *in vivo* antitumor effect of CUGACUC-containing siRNA. The ordinate shows a tumor size (mm³).
Figure 6 is a diagram showing the *in vivo* antitumor effect of CUGACUC-containing siRNA.
Figure 7 is a diagram showing the comparison of the ability of CnGACnC-containing siNA to suppress the expression of various genes. The ordinate shows a relative value of the expression level of various genes normalized with the expression level of GSTP1 with a value for a control group (Compound Z) defined as 100%. Compound names are indicated by only symbols.
Figure 8 is a picture showing the influence of CnGACnC-containing siNA on the survival of cells. Compound names are indicated by only symbols.
Figure 9 is a graph showing the influence of CnGACnC-containing siNA on the survival of cells. The ordinate shows a relative value of a viability with the expression level of GSTP1 for a control group (Compound Z) defined as 100%. Compound names are indicated by only symbols.

### DESCRIPTION OF EMBODIMENTS

All technical terms and scientific terms used herein have the same meanings as those usually understood by those skilled in the art, unless otherwise specified herein. All patents, applications and other publications (including online information) cited herein are incorporated herein by reference in their entirety.

The present specification encompasses the contents described in the specification and drawings of the Japanese application filed on March 28, 2019 (Japanese Application No. 2019-064443), based on which the priority of the present applications is claimed.

In one aspect, the present disclosure relates to an RNAi molecule comprising the nucleotide sequence of SEQ ID NO: 1 (CnGACnC) in an antisense strand (or an antisense region) (hereinafter, also referred to as the "RNAi molecule of the present disclosure").

The nucleotide sequence of SEQ ID NO: 1 consists of six RNAs and one DNA. In "CnGACnC", n represents U (uracil) or t (thymine), and C, G or A represents RNA or DNA. Specifically, the nucleotide sequence of SEQ ID NO: 1 includes the following seven nucleotide sequences: SEQ ID NO: 2 (cUGACUC), SEQ ID NO: 3(CtGACUC), SEQ ID NO: 4(CUgACUC), SEQ ID NO: 5(CUGaCUC), SEQ ID NO: 6(CUGAcUC), SEQ ID NO: 7(CUGACtC) and SEQ ID NO: 8(CUGACUc). Among these nucleotide sequences, SEQ ID NOs: 5 to 7 are preferred, SEQ ID NOs: 5 and 6 are more preferred, and SEQ ID NO: 5 is particularly preferred.

In the present specification, the upper-case letters in nucleotide sequences represent RNA, and the lower-case letters therein represent DNA, unless otherwise specified.

The RNAi molecule refers to any molecule capable of causing RNA interference. The RNA interference typically refers to a phenomenon, induced by a double-stranded nucleic acid molecule, in which target RNA is degraded in a sequence-specific manner. Upon entrance into a cell, the double-stranded nucleic acid molecule is cleaved by dicer according to its length and then, one of the strands (referred to as antisense strand or guide strand) is incorporated into RNA-induced silencing complex (RISC) containing Argonaute (AGO) protein. RISC is guided by an antisense strand (guide strand) having a sequence complementary to target RNA so that the RISC recognizes and cleaves the target RNA. When the target RNA is mRNA, a protein, etc. encoded by the mRNA is no longer expressed (gene silencing).

Examples of the RNAi molecule of the present disclosure include, but are not limited to, siNA (small interfering nucleic acid) such as siRNA (small interfering RNA), and shRNA. SiNA typically refers to a small nucleic acid with an antisense strand having complementarity to a target sequence, and a sense strand having complementarity to the antisense strand, wherein both the strands at least partially form a duplex.

The antisense strand and the sense strand in siNA may each independently have a length of 15 to 49 nucleotides (e.g., a length of about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides), about 17 to 35 nucleotides, about 17 to 30 nucleotides, about 15 to 25 nucleotides, about 18 to 25 nucleotides, about 18 to 23 nucleotides, about 19 to 21 nucleotides, about 25 to 30 nucleotides, or about 26 to 28 nucleotides. The duplex region may have a length of about 15 to 49 nucleotides (e.g., a length of about 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 or 49 nucleotides), about 15 to 35 nucleotides, about 15 to 30 nucleotides, about 15 to 25 nucleotides, about 17 to 25 nucleotides, about 17 to 23 nucleotides, about 17 to 21 nucleotides, about 25 to 30 nucleotides, or about 25 to 28 nucleotides.

In some embodiments, the sense strand and the antisense strand of siNA are separate polynucleotide strands. In such embodiments, the antisense strand and the sense strand may form a double-stranded structure via a hydrogen bond, for example, Watson-Crick base pairing, or through noncovalent linkage to each other. In other embodiments, the sense strand and the antisense strand constitute a portion of a single polynucleotide strand having a sense region and an antisense region. In such embodiments, the polynucleotide strand may have a hairpin structure.

siNA may have a blunt end or a protruding end. The protruding end may have an overhang of about 1, 2, 3, 4, 5, 6, 7 or 8 nucleotides. The overhang may be present at either the 5' end or the 3' end of the antisense strand and/or the sense strand or may be present at both the 5' end and the 3' end of the antisense strand or the sense strand. Specifically, the overhang may be present at the 5' end of the antisense strand, the 3' end of the antisense strand, both the 5' end and the 3' end of the antisense strand, the 5' end of the sense strand, the 3' end of the sense strand, both the 5' end and the 3' end of the sense strand, both the 5' end of the antisense strand and the 5' end of the sense strand, or both the 3' end of the antisense strand and the 3' end of the sense strand. The ends of siNA may be symmetric or asymmetric. Examples of the siNA having symmetric ends (hereinafter, also referred to as "symmetric siNA") include siNA having a blunt end as each end, and siNA having overhangs on the same sides of the antisense strand and the sense strand (e.g., siNA having overhangs with the same numbers of nucleotides at both the 5' end of the antisense strand and the 5' end of the sense strand, and siNA having overhangs with the same numbers of nucleotides at both the 3' end of the antisense strand and the 3' end of the sense strand). Examples of the siNA having asymmetric ends (hereinafter, also referred to as "asymmetric siNA") include siNA having a blunt end as one of the ends and a protruding end as the other end, and siNA having protruding ends as both ends which however differ in the position, length and/or type of an overhang. Examples of the siNA having asymmetric ends, both of which are protruding ends include siNA having overhangs at both the 5' end and the 3' end of the antisense strand or the sense strand, and siNA having overhangs on the same sides (i.e., the 5' ends or the 3' ends) of the antisense strand and the sense strand which however differ in the length and/or type of an overhang. The difference in the type of an overhang means, for example, difference in the types of nucleotides constituting the overhang. The nucleotides constituting the overhang include RNA, DNA, and nucleic acids having various modifications mentioned later. Thus, an overhang constituted by only unmodified RNA differs in type from an overhang comprising modified RNA, and an overhang constituted by certain modified RNA differs in type from an overhang constituted by another modified RNA.

In another embodiment, siNA may have a terminal loop structure. For example, siNA may have a hairpin structure having a loop structure at one of the ends and a blunt end as the other end (one polynucleotide has the sense strand and the antisense strand) or may have a hairpin structure having a loop structure at one of the ends and a protruding end as the other end (e.g., having an overhang of about 1, 2, 3, 4, 5, 6, 7 or 8 nucleotides). In the latter case, the overhang may be a 3' overhang or a 5' overhang, and the overhang may be present in the sense strand or the antisense strand.

In some embodiments, the sense strand of siNA may comprise one or more nicks. In such embodiments, the sense strand is divided by the nick. Upon incorporation of the antisense strand into RISC, the sense strand is separated into fragments at the position of the nick.

SiNA may comprise an unmodified nucleotide and/or a modified nucleotide. In the present specification, the unmodified nucleotide and the modified nucleotide are simply referred to as a "nucleotide" collectively. The unmodified nucleotide refers to a naturally occurring nucleotide constituting DNA or RNA, i.e., the one constituted by a nucleobase (adenine, guanine, uracil, thymine, or cytosine), a sugar (ribose or deoxyribose), and a phosphate group. In an unmodified nucleic acid molecule constituted by unmodified nucleotides, the 3' position of one of two adjacent unmodified nucleotides is usually linked to the 5' position of the other unmodified nucleotide through a phosphodiester bond. In one embodiment, the unmodified nucleotide is an unmodified ribonucleotide, and the unmodified nucleic acid molecule is constituted by unmodified ribonucleotides.

The modified nucleotide refers to a nucleotide containing a chemical modification to the unmodified nucleotide. The modified nucleotide may be artificially synthesized or may occur naturally. The modified nucleotide includes a nucleotide modified at its nucleobase, sugar, backbone (internucleotide bond), 5' end and/or 3' end. The modified nucleotide also includes a nucleotide modified at any one of these sites as well as a nucleotide modified at two or more of the sites.

Examples of the modification to the nucleobase include, but are not limited to, 2,4-difluorotoluyl, 2,6-diamino, 5-bromo, 5-iodo, 2-thio, dihydro, 5-propynyl, and 5-methyl modifications, and elimination of a base. Examples of modified nucleobase include, but are not limited to, xanthine, hypoxanthine, inosine, 2-aminoadenine, 6-methyl and other alkyl derivatives of adenine and guanine, universal base, 2-propyl and other alkyl derivatives of adenine and guanine, 5-halouracil and 5-halocytosine, 5-propynyl uracil and 5-propynyl cytosine, 6-azo uracil, 6-azo cytosine and 6-azo thymine, 5-uracil (pseudouracil), 4-thiouracil, 8-halo, amino, thiol, thioalkyl, hydroxyl and other 8-substituted adenines and guanines, 5-trifluoromethyl and other 5-substituted uracils and cytosines, 7-methylguanine, acyclonucleotides, deazapurines, heterocyclic substituted analogs of purines and pyrimidines, e.g., aminoethyoxy phenoxazine, derivatives of purines and pyrimidines (e.g., 1-alkyl-, 1-alkenyl-, heteroaromatic- and 1-alkynyl derivatives) and tautomers thereof, 8-oxo-N6-methyladenine, 7-diazaxanthine, 5-methylcytosine, 5-methyluracil, 5-(1-propynyl) uracil, 5-(1-propynyl) cytosine and 4,4-ethanocytosine, non-purinyl and non-pyrimidinyl bases such as 2-aminopyridine and triazines, abasic nucleotide, deoxy abasic nucleotide, inverted abasic nucleotide, inverted deoxy abasic nucleotide, and the like.

Examples of the modification to the sugar include, but are not limited to: modifications at position 2', for example, 2'-O-alkyl modifications (e.g., 2'-O-methyl modification and 2'-O-ethyl modification), 2'-methoxyethoxy modification, 2'-methoxyethyl modification, 2'-deoxy modification, 2'-halogen modifications (2'-fluoro modification, 2'-chloro modification, 2'-bromo modification, etc.), 2'-O-allyl modification, 2'-amino modification, 2'-S-alkyl modification, 2'-O-[2(methylamino)-2-oxoethyl] modification, 2'-alkoxy modification, 2'-O-2-methoxyethyl, 2'-allyloxy (-OCH₂CH=CH₂), 2'-propargyl, 2'-propyl, 2'-O-(N-methyl carbamate) modification, 2'-O-(2,4-dinitrophenyl) modification, and 2'-deoxy-2'-fluoro-β-D-arabino modification; modifications at position 4', for example, 4'-thio modification and 4'-C-hydroxymethyl modification; and other modifications with ethynyl, ethenyl, propenyl, CF, cyano, imidazole, carboxylate, thioate, C₁ to C₁₀ lower alkyl, substituted lower alkyl, alkaryl or aralkyl, OCF₃, OCN, O-, S- or N-alkyl, O-, S- or N-alkenyl, SOCH₃, SO₂CH₃, ONO₂, NO₂, N₃, heterocycloalkyl, heterocycloalkaryl, aminoalkylamino, polyalkylamino or substituted silyl. Other examples of the modified sugar include locked nucleic acid (LNA), oxetane-LNA (OXE), unlocked nucleic acid (UNA), ethylene-bridged nucleic acid (ENA), altriol nucleic acid (ANA), and hexitol nucleic acid (HNA).

In the present disclosure, alkyl group includes saturated aliphatic groups, including straight-chain alkyl groups (e.g., methyl, ethyl, propyl, butyl, pentyl, hexyl, heptyl, octyl, nonyl, decyl, etc.), branched-chain alkyl groups (isopropyl, tert-butyl, isobutyl, etc.), cycloalkyl (alicyclic) groups (cyclopropyl, cyclopentyl, cyclohexyl, cycloheptyl, cyclooctyl), and alkyl substituted cycloalkyl groups. In certain embodiments, a straight chain or branched chain alkyl has 6 or fewer carbon atoms in its backbone (e.g., C₁-C₆ for straight chain, C₃-C₆ for branched chain), and more preferably 4 or fewer. Likewise, preferred cycloalkyls may have from 3-8 carbon atoms in their ring structure, and more preferably have 5 or 6 carbons in the ring structure. The term C₁-C₆ includes alkyl groups containing 1 to 6 carbon atoms. The alkyl group can be substituted alkyl group such as alkyl moieties having substituents replacing a hydrogen on one or more carbons of the hydrocarbon backbone. Such substituents can include, for example, alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moiety.

In the present disclosure, alkoxy group includes substituted and unsubstituted alkyl, alkenyl, and alkynyl groups covalently linked to an oxygen atom. Examples of alkoxy groups include methoxy, ethoxy, isopropyloxy, propoxy, butoxy, and pentoxy groups. Examples of substituted alkoxy groups include halogenated alkoxy groups. The alkoxy groups can be substituted with groups such as alkenyl, alkynyl, halogen, hydroxyl, alkylcarbonyloxy, arylcarbonyloxy, alkoxycarbonyloxy, aryloxycarbonyloxy, carboxylate, alkylcarbonyl, arylcarbonyl, alkoxycarbonyl, aminocarbonyl, alkylaminocarbonyl, dialkylaminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, alkylsulfinyl, sulfonato, sulfamoyl, sulfonamido, nitro, trifluoromethyl, cyano, azido, heterocyclyl, alkylaryl, or an aromatic or heteroaromatic moieties. Examples of halogen substituted alkoxy groups include, but are not limited to, fluoromethoxy, difluoromethoxy, trifluoromethoxy, chloromethoxy, dichloromethoxy, trichloromethoxy, etc.

In the present disclosure, halogens include fluorine, bromine, chlorine, iodine.

Examples of modified backbone include, but are not limited to phosphorothioate, thiophosphate-D-ribose entities, triester, thioate, 2'-5' bridged backbone (may also be referred to as 5'-2' or 2'5' nucleotide or 2'5' ribonucleotide), PACE, 3'-(or -5')deoxy-3'-(or -5')thio-phosphorothioate, phosphorodithioate, phosphoroselenates, 3'-(or -5')deoxy phosphinates, borano phosphates, 3'-(or - 5')deoxy-3'-(or 5'-)amino phosphoramidates, hydrogen phosphonates, phosphonates, borano phosphate esters, phosphoramidates, alkyl or aryl phosphonates and phosphotriester modifications such as alkylphosphotriesters, phosphotriester phosphorus linkages, 5'-ethoxyphosphodiester, P-alkyloxyphosphotriester, methylphosphonate and morpholino, and nonphosphorus containing linkages for example, carbonate, carbamate, silyl, sulfur, sulfonate, sulfonamide, formacetal, thioformacetyl, oxime, methyleneimino, methylenemethylimino, methylenehydrazo, methylenedimethylhydrazo and methyleneoxymethylimino linkages.

Examples of 5'- and/or 3'- end modification include addition of a capping moiety to 5'- and/or 3'- end, and modification at terminal phosphate groups, such as [3-3']-inverted deoxyribose, deoxyribonucleotide, [5'-3']-3'-deoxyribonucleotide, [5'-3']-ribonucleotide, [5'-3']-3'-O-methyl ribonucleotide, 3'-glyceryl, [3'-5']-3'-deoxyribonucleotide, [3'-3']-deoxyribonucleotide, [5'-2']-deoxyribonucleotide, and [5-3']-dideoxyribonucleotide. Non-limiting examples of capping moiety include an abasic nucleotide, a deoxy abasic nucleotide, an inverted (deoxy) abasic nucleotide, a hydrocarbon (alkyl) moiety and derivatives thereof, a mirror nucleotide (L-DNA or L-RNA), bridged nucleic acids including LNA and ethylene bridged nucleic acids, linkage modified nucleotides (e.g. PACE) and base modified nucleotides, glyceryl, dinucleotide, acyclic nucleotide, amino, fluoro, chloro, bromo, CN, CF, methoxy, imidazole, carboxylate, thioate, C₁ to C₁₀ lower alkyl, substituted lower alkyl, alkaryl or aralkyl, OCF₃, OCN, O , S , or N-alkyl, O , S , or N-alkenyl, SOCH₃, SO₂CH₃, ONO₂, NO₂, N₃, heterocycloalkyl, heterocycloalkaryl, aminoalkylamino, polyalkylamino or substituted silyl. The capping moiety may serve as a non-nucleotide overhang.

Modified nucleotides of the present disclosure include 2'-deoxyribonucleotides, 2'-O-methyl ribonucleotides, 2'-deoxy-2'-fluoro ribonucleotides, universal base nucleotides, acyclic nucleotides, 5-C-methyl nucleotides, nucleotides containing biotin group, and terminal glyceryl and/or inverted deoxy abasic residue, nucleotide containing sterically hindered molecules, such as fluorescent molecules and the like, 3'-deoxyadenosine (cordycepin), 3'-azido-3'-deoxythymidine (AZT), 2',3'-dideoxyinosine (ddI), 2',3'-dideoxy-3'-thiacytidine (3TC), 2',3'-didehydro-2',3'-dideoxythymidine (d4T), nucleotides containing 3'-azido-3'-deoxythymidine (AZT), 2',3'-dideoxy-3'-thiacytidine (3TC) or 2',3'-didehydro-2',3'-dide-oxythymidine (d4T), a nucleotide having a Northern conformation, 2'-methyl-thio-ethyl, 2'-deoxy-2'-fluoro nucleotides, 2'-deoxy-2'-chloro nucleotides, 2'-azido nucleotides, and 2'-O-methyl nucleotides, 6-membered ring nucleotide analogs including hexitol and altritol nucleotide monomers disclosed in WO 2006/047842, etc., mirror nucleotides (for example L-DNA (L-deoxyriboadenosine-3'-phosphate (mirror dA), L-deoxyribocytidine-3'-phosphate (mirror dC), L-deoxyriboguanosine-3'-phosphate (mirror dG), L-deoxyribothymidine-3'-phosphate (mirror image dT)) and L-RNA (L-riboadenosine-3'-phosphate (mirror rA), L-ribocytidine-3'-phosphate (mirror rC), L-riboguanosine-3'-phosphate (mirror rG), L-ribouracil-3'-phosphate (mirror dU), etc.).

Non-limiting examples of the modified nucleotide are also described in, for example, Gaglione and Messere, Mini Rev Med Chem. 2010; 10 (7): 578-95, Deleavey and Damha, Chem Biol. 2012; 19 (8): 937-54, and Bramsen and Kjems, J. Front Genet. 2012; 3: 154.

In one embodiment, the CnGACnC sequence contained in the antisense strand consists of RNA and DNA. In a particular embodiment, the CnGACnC sequence contained in the antisense strand consists of unmodified RNA and DNA.

shRNA is a single-stranded RNA molecule having an antisense region and a sense region having complementarity to each other, and a loop region interposed therebetween, and assumes a hairpin-shaped three-dimensional structure formed from a duplex region by the pairing of the antisense region and the sense region. shRNA according to the present disclosure comprises at least one DNA in the molecule.

When the RNAi molecule of the present disclosure is shRNA, the description regarding the antisense strand in siNA is applied to the antisense region of shRNA and the description regarding the sense strand in siNA is applied to the sense region of shRNA. Thus, the shRNA of the present disclosure comprises the CnGACnC sequence in the antisense region. The antisense region and the sense region in the shRNA of the present disclosure may each independently have a length of about 15 to 49 nucleotides, about 17 to 35 nucleotides, about 17 to 30 nucleotides, about 15 to 25 nucleotides, about 18 to 25 nucleotides, about 18 to 23 nucleotides, about 19 to 21 nucleotides, about 25 to 30 nucleotides, or about 26 to 28 nucleotides. The duplex region may have a length of about 15 to 49 nucleotides, about 15 to 35 nucleotides, about 15 to 30 nucleotides, about 15 to 25 nucleotides, about 17 to 25 nucleotides, about 17 to 23 nucleotides, about 17 to 21 nucleotides, about 25 to 30 nucleotides, or about 25 to 28 nucleotides. The length of the loop region is not particularly limited as far as it is possible to be cleaved by dicer, and may be about 2 to 100 nucleotides, about 3 to 80 nucleotides, about 4 to 70 nucleotides, about 5 to 60 nucleotides, about 6 to 50 nucleotides, or the like.

In one embodiment, the RNAi molecule of the present disclosure suppresses the protein expression of a member of the BcL2 family. In a preferred embodiment, the BcL2 family is an antiapoptotic BcL2 family. The antiapoptotic BcL2 family includes BcL-2, BcL-XL, BFL1, BcL-W, and the like. In a particularly preferred embodiment, the BcL2 family molecule is BcL-XL. The antiapoptotic BcL2 family molecule is considered to suppress apoptosis through interaction with a proapoptotic BcL2 family molecule (e.g., multidomain proteins such as BAX, BOK, and BAK, and BH3-only proteins such as BIM, BAD, BID, NOXA, PUMA (Bbc3), BMF, HRK, and BIK). In a particular embodiment, the RNAi molecule of the present disclosure suppresses the expression of BcL-XL.

In a more preferred embodiment, the RNAi molecule of the present disclosure suppresses the expression of one or more genes selected at least from MRS2, RFC1, BcL-2, Smad1, P21, TJP2, SIKE1, GPANK1, HSPA12A and TYW3, in addition to BcL-XL. In a particular embodiment, the RNAi molecule of the present disclosure suppresses the expression of genes including at least the following combinations of genes: BcL-XL and MRS2; BcL-XL and RFC1; BcL-XL and BcL-2; BcL-XL and Smad1; BcL-XL and P21; BcL-XL and TJP2; BcL-XL and SIKE1; BcL-XL and GPANK1; BcL-XL and HSPA12A; BcL-XL and TYW3; BcL-XL, MRS2 and RFC1; BcL-XL, BcL-2 and Smad1; BcL-XL, BcL-2 and P21; BcL-XL, BcL-2 and MRS2; BcL-XL, Smad1 and P21; BcL-XL, Smad1 and MRS2; BcL-XL, P21 and MRS2; and BcL-XL, Smad1, P21 and MRS2.

The suppression of gene expression or protein expression can be evaluated, for example, by comparing the expression level of the gene or the protein between cells with the action of the RNAi molecule of the present disclosure and cells without the action thereof. The expression level of the gene can be determined by a known detection approach, for example, by detecting a nucleic acid molecule encoding the gene via various hybridization methods, Northern blotting, Southern blotting, or various PCR methods using a nucleic acid specifically hybridizing to the nucleic acid molecule or a unique fragment thereof. The expression level of the protein can be determined by a known protein detection approach, for example, an immunoprecipitation method using an antibody, EIA (enzyme immunoassay) (e.g., ELISA (enzyme-linked immunosorbent assay)), RIA (radioimmunoassay) (e.g., IRMA (immunoradiometric assay), RAST (radioallergosorbent test), and RIST (radioimmunosorbent test)), Western blotting, an immunohistochemical method, an immunocytochemical method, or flow cytometry, without limitations.

In one embodiment, the RNAi molecule of the present disclosure targets BcL2L1 encoding BcL-XL. The sequence of BcL2L1 is known in the art. The sequence of human BcL2L1 mRNA has been registered, for example, under accession numbers NM_138578.3 (SEQ ID NO: 9), NM 001317919.1 (SEQ ID NO: 10), NM001317920.1 (SEQ ID NO: 11), NM_001317921.1 (SEQ ID NO: 12), NM_001322239.1 (SEQ ID NO: 13), NM_001322240.1 (SEQ ID NO: 14), and NM_001322242.1 (SEQ ID NO: 15). Since the antisense strand of the RNAi molecule of the present disclosure comprises the nucleotide sequence of SEQ ID NO: 1, the RNAi molecule of the present disclosure typically targets a region comprising a sequence (GAGTCAG, SEQ ID NO: 16) having complementarity to SEQ ID NO: 1 in BcL2L1. The term "having complementarity" or "being complementary" means that a certain nucleic acid molecule can form a classic Watson-Crick-type or non-classic-type hydrogen bond with another nucleic acid molecule. The term "complementarity percent" refers to the percentage of nucleotides of a certain nucleic acid molecule that can form a hydrogen bond (e.g., a Watson-Crick base pair) with nucleotides of another nucleic acid molecule. For example, when 5, 6, 7, 8, 9 or 10 out of a total of 10 nucleotides in a first oligonucleotide form a base pair with a second oligonucleotide having 10 nucleotides, the complementarity percent is 50%, 60%, 70%, 80%, 90% or 100%, respectively. The term "being completely complementary" or "having complete complementarity" means that all the nucleotides of a certain nucleic acid molecule form a hydrogen bond with the same number thereas of consecutive nucleotides in another nucleic acid molecule. In one embodiment, the antisense strand of the RNAi molecule of the present disclosure is completely complementary to a target nucleic acid molecule or a portion thereof.

In a preferred embodiment, the RNAi molecule of the present disclosure has the nucleotide sequence of SEQ ID NO: 1 at positions 2 to 8 from the 5' end of the antisense strand (or the antisense region). In a particular preferred embodiment, the RNAi molecule of the present disclosure has an antisense strand (or an antisense region) comprising any of sequences given below. The target site represents a position in NM_138578.3 (SEQ ID NO: 9).

**Table 1 Exemplary sequences of antisense strand**

| SEQ ID NO: | Sequence (5' to 3') | Length | Target site |
|---|---|---|---|
| 17 | ACnGACnCCAGCUGU | 15 | 446-460 |
| 18 | ACnGACnCCAGCUGUA | 16 | 445-460 |
| 19 | ACnGACnCCAGCUGUAU | 17 | 444-460 |
| 20 | ACnGACnCCAGCUGUAUC | 18 | 443-460 |
| 21 | ACnGACnCCAGCUGUAUCC | 19 | 442-460 |
| 22 | ACnGACnCCAGCUGUAUCCU | 20 | 441-460 |
| 23 | ACnGACnCCAGCUGUAUCCUU | 21 | 440-460 |
| 24 | ACnGACnCCAGCUGUAUCCUUU | 22 | 439-460 |
| 25 | ACnGACnCCAGCUGUAUCCUUUC | 23 | 438-460 |
| 26 | ACnGACnCCAGCUGUAUCCUUUCU | 24 | 437-460 |
| 27 | ACnGACnCCAGCUGUAUCCUUUCUG | 25 | 436-460 |
| 28 | ACnGACnCCAGCUGUAUCCUUUCUGG | 26 | 435-460 |
| 29 | ACnGACnCCAGCUGUAUCCUUUCUGGG | 27 | 434-460 |
| 30 | ACnGACnCCAGCUGUAUCCUUUCUGGGA | 28 | 433-460 |
| 31 | ACnGACnCCAGCUGUAUCCUUUCUGGGAA | 29 | 432-460 |
| 32 | ACnGACnCCAGCUGUAUCCUUUCUGGGAAA | 30 | 431-460 |
| 33 | ACnGACnCCAGCUGUAUCCUUUCUGGGAAAG | 31 | 430-460 |
| 34 | ACnGACnCCAGCUGUAUCCUUUCUGGGAAAGC | 32 | 429-460 |
| 35 | ACnGACnCCAGCUGUAUCCUUUCUGGGAAAGCU | 33 | 428-460 |
| 36 | ACnGACnCCAGCUGUAUCCUUUCUGGGAAAGCUU | 34 | 427-460 |
| 37 | ACnGACnCCAGCUGUAUCCUUUCUGGGAAAGCUUG | 35 | 426-460 |

The RNAi molecule of the present disclosure may be delivered or administered together with any known delivery carrier having an effect of assisting in, promoting or facilitating delivery to a site of action, or may be delivered or administered directly without such a delivery carrier. A viral vector or a non-viral vector can be used as the delivery carrier.

Examples of the viral vector include, but are not limited to, vectors based on adenovirus, adeno-associated virus (AAV), retrovirus, vaccinia virus, poxvirus, lentivirus, and herpes virus. The viral vector may be oncolytic. The oncolytic viral vector is particularly useful for the treatment of a cancer.

Examples of the non-viral vector include, but are not limited to, carriers in a particle form such as polymer particles, lipid particles and inorganic particles, and a bacterial vector. Nanoparticles having a nano level of size can be used as the carrier in a particle form. Examples of the polymer particles include, but are not limited to, those comprising polymers such as cationic polymers, polyamidoamine (PAMAM), chitosan, cyclodextrin, poly(lactic-co-glycolic acid) (PLGA), poly(lacticco-caprolactonic acid) (PLCA), pοly(β amino ester), and atelocollagen. The lipid particles include liposomes, non-liposomal lipid particles, and the like. The liposome is a vesicle having a lumen surrounded by a lipid bilayer, and the non-liposomal lipid particles are lipid particles having no such structure. Examples of the inorganic particles include gold nanoparticles, quantum dots, silica nanoparticles, iron oxide nanoparticles (e.g., superparamagnetic iron oxide nanoparticles (SPION)), nanotubes (e.g., carbon nanotubes (CNT)), nanodiamond, and fullerene. Examples of the bacterial vector include, but are not limited to, vectors based on *Listeria* bacterium, bifidus, and salmonella.

The RNAi molecule of the present disclosure can be systemically administered or locally administered *ex vivo* or *in vivo* to a tissue concerned via skin application, transdermal application or injection (intravenous injection, intradermal injection, subcutaneous injection, intramuscular injection, intraarterial injection, drip injection, etc.).

The RNAi molecule of the present disclosure can be delivered through a delivery system suitable for a purpose. The delivery system may include, for example, aqueous and non-aqueous gels, creams, double emulsions, microemulsions, liposomes, ointments, aqueous and non-aqueous solutions, lotions, aerosols, hydrocarbon bases and powders and can comprise excipients, for example, solubilizers, penetration enhancers (e.g., fatty acids, fatty acid esters, aliphatic alcohols and amino acids) and hydrophilic polymers (e.g., polycarbophil and polyvinylpyrrolidone). In one embodiment, the pharmaceutically acceptable carrier is a liposome or a transdermal delivery enhancer.

The delivery system may include patches, tablets, suppositories, pessaries, gels and creams and can comprise excipients, for example, solubilizers and enhancers (e.g., propylene glycol, bile salt and amino acids) and other vehicles (e.g., polyethylene glycol, fatty acid esters and derivatives, and hydrophilic polymers, for example, hydroxypropylmethylcellulose and hyaluronic acid).

Approaches and systems useful in the delivery of the RNAi molecule of the present disclosure are described in, for example, Rettig and Behlke, Mol Ther. 2012; 20 (3): 483-512, Kraft et al., J Pharm Sci. 2014; 103 (1): 29-52, Hong and Nam, Theranostics. 2014; 4 (12): 1211-32, and Kaczmarek et al., Genome Med. 2017; 9 (1): 60.

In some embodiments, the present disclosure relates to a composition comprising the RNAi molecule of the present disclosure (hereinafter, also referred to as the composition of the present disclosure). The composition of the present disclosure may comprise any carrier, diluent, delivery vehicle, delivery system, and the like, mentioned above, in addition to the RNAi molecule of the present disclosure. The composition of the present disclosure can be used in the treatment of a disease such as a cancer. Thus, the composition of the present disclosure may serve as a pharmaceutical composition for the treatment of a disease such as a cancer (hereinafter, also referred to as the pharmaceutical composition of the present disclosure). The pharmaceutical composition of the present disclosure may comprise one or more pharmaceutically acceptable additives (e.g., surfactants, carriers, diluents, and excipients). The pharmaceutically acceptable additives are well known in the medical field and described in, for example, Remington's Pharmaceutical Sciences, 18th Ed., Mack Publishing Co., Easton, PA (1990), which is incorporated herein by reference in its entirety.

In some embodiments, the RNAi molecule and the pharmaceutical composition of the present disclosure can be used for the treatment of a disease that can be improved by the suppression of BcL-XL includes, for example, cell proliferative diseases. Non-limiting examples of the cell proliferative disease include cancers, lymphoproliferative diseases, polycythemia rubra vera, pulmonary hypertension, hyperplasia, keloid, Cushing's syndrome, primary aldosteronism, erythroplakia, leukoplakia, hyperplastic scar, lichen planus and lentiginosis. The cancer includes epithelial malignant tumor and non-epithelial malignant tumor. The cancer includes epithelial malignant tumor and non-epithelial malignant tumor. The cancer to be treated includes, but not limited to, for example, brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia. The cancer may be present in any site, for example, the brain, the head and the neck, the chest, extremities, the lung, the heart, thymus gland, the esophagus, the stomach, the small intestine (the duodenum, the jejunum, and the ileum), the large intestine (the colon, the cecum, the appendix, and the rectum), the liver, the pancreas, the gallbladder, the anus, the kidney, the ureter, the bladder, the prostate, the penis, the testis, the uterus, the ovary, the vulva, the vagina, the skin, striated muscle, smooth muscle, synovium, cartilage, bone, thyroid gland, adrenal gland, peritoneum, mesentery, bone marrow, blood, the vascular system, the lymphatic system such as lymph nodes, and lymph.

In a particular embodiment, the RNAi molecule and the pharmaceutical composition of the present disclosure can be used for the treatment of a disease associated with a proliferation of a cell expressing BcL-XL, e.g., for the treatment of a cancer expressing BcL-XL. In a preferred embodiment, BcL-XL in the cell or the cancer is overexpressed. Whether or not a certain cell or cancer expresses BcL-XL or whether or not a certain cell or cancer overexpresses BcL-XL is known from a literature or the like or can be determined, for example, by detecting the expression of BcL-XL in the cell or in cancer cells constituting the cancer. The expression of BcL-XL can be determined by a known detection approach, for example, by detecting a nucleic acid molecule encoding BcL-XL (BcL2L1) via various hybridization methods, Northern blotting, Southern blotting, or various PCR methods using a nucleic acid specifically hybridizing to the nucleic acid molecule or a unique fragment thereof, or by detecting BcL-XL via a known protein detection approach, for example, an immunoprecipitation method using an antibody, EIA (e.g., ELISA), RIA (e.g., IRMA, RAST, and RIST), Western blotting, an immunohistochemical method, an immunocytochemical method, or flow cytometry, without limitations. Since the overexpression of BcL-XL in cancer cells may be caused by the amplification of BcL2L1 gene, the amplification of the BcL2L1 gene can be used as an index for the overexpression of BcL-XL. It has been reported that the amplification of the BcL2L1 gene is found in bladder cancer, breast cancer, head and neck cancer, lung cancer, stomach cancer, uterus cancer, and the like (Campbell and Tait, Open Biol. 2018; 8 (5): 180002).

The cell or cancer to be treated by the RNAi molecule of the present disclosure preferably expresses, besides BcL-XL, one or more genes selected from MRS2, RFC1, BcL-2, Smad1, P21, TJP2, SIKE1, GPANK1, HSPA12A and TYW3. In a particular embodiment, the cell or cancer to be treated by the RNAi molecule of the present disclosure expresses genes including at least the following combinations of genes: BcL-XL and MRS2; BcL-XL and RFC1; BcL-XL and BcL-2; BcL-XL and Smad1; BcL-XL and P21; BcL-XL and TJP2; BcL-XL and SIKE1; BcL-XL and GPANK1; BcL-XL and HSPA12A; BcL-XL and TYW3; BcL-XL, MRS2 and RFC1; BcL-XL, BcL-2 and Smad1; BcL-XL, BcL-2 and P21; BcL-XL, BcL-2 and MRS2; BcL-XL, Smad1 and P21; BcL-XL, Smad1 and MRS2; BcL-XL, P21 and MRS2; and BcL-XL, Smad1, P21 and MRS2.

In the present disclosure, the "treatment" includes every type of medically acceptable prophylactic and/or therapeutic intervention aimed at cure, transient remission or prevention, etc. of a disease. The "treatment" includes medically acceptable intervention for various purposes including, for example, the delay or arrest of progression of a disease, the regression or disappearance of a lesion, the prevention of onset of the disease or the prevention of recurrence of the disease. Thus, the RNAi molecule and the pharmaceutical composition can be used in the treatment and/or the prevention of a disease.

The RNAi molecule and the pharmaceutical composition of the present disclosure can also be used for the treatment of a disease caused by abnormality in apoptosis, for example, a disease caused by the abnormal proliferation of cells. Examples of the disease caused by the abnormal proliferation of cells include, but are not limited to, benign or malignant tumor, hyperplasia, keloid, Cushing's syndrome, primary aldosteronism, erythroplakia, polycythemia rubra vera, pulmonary hypertension, leukoplakia, hyperplastic scar, lichen planus and lentiginosis.

The RNAi molecule and the pharmaceutical composition of the present disclosure can also be used for the treatment of a disease caused by the expression of BcL-XL, for example, a disease caused by the abnormal proliferation of cells associated with the expression of BcL-XL. Examples of the disease caused by the abnormal proliferation of cells include, but are not limited to, benign or malignant tumor and lymphoproliferative diseases.

The RNAi molecule or the pharmaceutical composition of the present disclosure may be administered through various routes including both oral and parenteral routes, for example, but not limited to, oral, buccal, mouth, intravenous, intramuscular, subcutaneous, intradermal, local, rectal, intraarterial, intraportal, intraventricular, transmucosal, transdermal, intranasal, intraperitoneal, intratracheal, intrapulmonary and intrauterine routes, and may be formulated into a dosage form suitable for each administration route. As such a dosage form and a formulation method, any ones known in the art can be appropriately adopted (see e.g., Remington's Pharmaceutical Sciences, 18th Ed., Mack Publishing Co., Easton, PA (1990)).

Examples of the dosage form suitable for oral administration include, but are not limited to, powders, granules, tablets, capsules, solutions, suspensions, emulsions, gels, and syrups. Examples of the dosage form suitable for parenteral administration include injections such as solution-type injections, suspension-type injections, emulsion-type injection, and injections to be prepared at the time of use. The preparation for parenteral administration can be in the form of an aqueous or non-aqueous isotonic sterile solution or suspension.

The composition according to the present disclosure may be supplied in any form and may be provided in a form capable of being prepared at the time of use, for example, a form capable of being prepared by a physician and/or a pharmacist, a nurse, or other paramedical staff, etc. in or near medical setting, from the viewpoint of preservation stability. In this case, the composition is provided in one or more containers comprising at least one component essential therefor, and prepared before use, for example, within 24 hours before use, preferably within 3 hours before use, more preferably immediately before use. For the preparation, a reagent, a solvent, pharmacy equipment, and the like usually available in a place of preparation can be appropriately used.

In further aspects, the present disclosure relates to a kit or a pack for preparing the RNAi molecule or the composition and/or for treating a disease, comprising the RNAi molecule or the composition according to the present disclosure or a component thereof, and the RNAi molecule or the composition, or a necessary component thereof that is provided in the form of such a kit or a pack. Each component of the RNAi molecule or the composition contained in this kit or pack is as described above with respect to the RNAi molecule or the composition. The present kit may further comprise instructions as to a method for preparing or using (e.g., administering) the RNAi molecule or the composition, for example, instruction manuals, and a medium, for example, a flexible disc, CD, DVD, a Blu-ray disc, a memory card, or a USB memory, in which information on the use method is recorded, in addition to those described above. Also, the kit or the pack may comprise all components for completing the RNAi molecule or the composition or may not necessarily comprise all the components. Thus, the kit or the pack may not comprise a reagent or a solvent usually available in a medical setting, an experimental facility, etc., for example, sterile water, saline, or a glucose solution.

In an alternative aspect, the present disclosure relates to a method for treating a disease that can be improved by the suppression of BcL-XL, a disease caused by abnormal apoptosis or a disease caused by the expression of BcL-XL, the method comprising the step of administering an effective amount of the RNAi molecule or the pharmaceutical composition according to the present disclosure to a subject in need thereof (hereinafter, also referred to as the "treatment method of the present disclosure"). In this context, the effective amount is, for example, an amount in which the onset and recurrence of the disease are prevented, or the disease is cured. Each of the terms "disease that can be improved by the suppression of BcL-XL", "disease caused by abnormality in apoptosis", "disease caused by the expression of BcL-XL", and "treatment" for the treatment method of the present disclosure is as described above with respect to the RNAi molecule of the present disclosure.

In the treatment method, the specific dose of the RNAi molecule or the pharmaceutical composition to be administered to the subject may be determined in consideration of various conditions as to the subject in need of the administration, for example, the type of the target, the purpose of the method, a therapeutic regimen, the type of the disease, the severity of symptoms, the general health state, age, and body weight of the subject, the sex of the subject, diets, the timing and frequency of administration, a concurrent drug, responsiveness to therapy, and compliance with therapy. The total daily dose of the RNAi molecule or the pharmaceutical composition is not limited and may be, for example, about 1 µg/kg to about 1000 mg/kg body weight, about 10 µg/kg to about 100 mg/kg body weight, or about 100 µg/kg to about 10 mg/kg body weight, in terms of the amount of the RNAi molecule. Alternatively, the dose may be calculated on the basis of the surface area of a patient.

The administration route includes various routes including both oral and parenteral routes, for example, oral, buccal, mouth, intravenous, intramuscular, subcutaneous, intradermal, local, rectal, intraarterial, intraportal, intraventricular, transmucosal, transdermal, intranasal, intraperitoneal, intratracheal, intrapulmonary and intrauterine routes.

The frequency of administration differs depending on the properties of the preparation or the composition used or the conditions of the subject as described above and may be, for example, plural times per day (i.e., 2, 3, 4 or 5 or more times per day), once a day, every few days (i.e., every 2, 3, 4, 5, 6, or 7 days), several times a week (e.g., 2, 3, or 4 times a week), every week, or every few weeks (i.e., every 2, 3, or 4 weeks).

In the present disclosure, the term "subject" means any individual organism, preferably animal, more preferably mammalian, further preferably human individual. The subject may be healthy (e.g., have no particular or any disease) or may be affected by some disease. When the treatment of a disease associated with a target nucleic acid molecule is intended, for example, the subject typically means a subject affected by the disease or having a risk of being affected by the disease.

In an alternative aspect, the present disclosure relates to use of the RNAi molecule of the present disclosure in the production of a medicament for the treatment of a disease that can be improved by the suppression of BcL-XL, a disease caused by abnormality in apoptosis and/or a disease caused by the expression of BcL-XL (hereinafter, also referred to as the "use of present disclosure").

Each of the terms "disease that can be improved by the suppression of BcL-XL", "disease caused by abnormality in apoptosis", "disease caused by the expression of BcL-XL", and "treatment" for the use of the present disclosure is as described above with respect to the RNAi molecule of the present disclosure.

### EXAMPLES

Some embodiments of the present disclosure will be described in more detail with reference to the examples given below. However, these examples are given for illustrative purposes and do not limit the scopes of the embodiments.

### Example 1: Testing of ability of siRNA targeting BcL-XL to suppress cancer cell proliferation

An experiment was conducted using siRNAs targeting BcL-XL given below.
Compound X (Comprising CUGACUC sequence in antisense strand)
Sense strand: 5' -GGAUACAGCUGGAGUCAGUtt-3' (SEQ ID NO: 38)
Antisense strand: 5' -ACUGACUCCAGCUGUAUCCtt-3' (SEQ ID NO: 39)
Compound Y (Comprising no CUGACUC sequence in antisense strand)
Sense strand: 5' -GGUAUUGGUGAGUCGGAUCtt-3' (SEQ ID NO: 40)
Antisense strand: 5' -GAUCCGACUCACCAAUACCtt-3' (SEQ ID NO: 41)
Compound Z (Control, Allstars negative control siRNA (Qiagen N.V.))

A colon cancer cell line HCT116, a breast cancer cell line MDA-MB-231, a skin cancer cell line A375, or a colon cancer cell line SW480 was cultured in McCoy's 5A medium (Sigma-Aldrich Co. LLC) for HCT116 or DMEM medium (Sigma-Aldrich Co. LLC) for MDA-MB-231, A375, and SW480 (each medium contained 10% inactivated fetal bovine serum (FBS), and 100 U/mL penicillin and 100 µg/mL streptomycin as antibiotics) under conditions of 37°C and 5% CO₂.

Transfection with siRNAs (Compounds X to Z) was performed as follows: on the day before transfection, HCT116 cells and A375 cells were seeded at 0.25 × 10⁵ cells/well, and MDA-MB-231 cells and SW480 cells were seeded at 0.5 × 10⁵ cells/well, to a 6-well tissue culture plastic dish. 27.5 pmol of siRNA was added into 125 µL of Opti-MEM I Reduced Serum Medium (Invitrogen Corp.) and gently mixed. Next, 3 µL of Lipofectamine RNAiMAX (Invitrogen Corp.) was diluted into 125 µL of Opti-MEM I Reduced Serum Medium and gently mixed. The siRNA dilution and the Lipofectamine RNAiMAX dilution were combined, gently mixed, and then incubated at room temperature for 15 minutes. During this period, the medium was replaced with 2.5 mL of Opti-MEM I Reduced Serum Medium. After the incubation for 15 minutes, the complex of siRNA and Lipofectamine RNAiMAX was added to the cells and incubated at 37°C in the atmosphere containing 5% CO₂. After the incubation for 5 hours, the medium was replaced with 3 mL of medium containing 10% FBS. On day 3 after the transfection, the cell number was counted. As shown in results in Figure 1, Compound X suppressed the proliferation of all the cancer cells more strongly than Compound Y did.

### Example 2: Testing of ability of siRNA targeting BcL-XL to suppress cancer cell proliferation and ability thereof to kill cancer cell

Cells of a lung cancer cell line A549, a pancreatic cancer cell line SUIT-2, or a pancreatic cancer cell line SW1990 were cultured in DMEM medium (Sigma-Aldrich Co. LLC) for A549, MEM medium (Sigma-Aldrich Co. LLC) for SUIT-2, and RPMI1640 (Sigma-Aldrich Co. LLC) for SW1990 (each medium contained 10% inactivated fetal bovine serum (FBS), and 100 U/mL penicillin and 100 µg/mL streptomycin as antibiotics) under conditions of 37°C and 5% CO₂.

Transfection with siRNAs (Compounds X to Z) was performed as follows: on the day before transfection, each cell was seeded to a 96-well tissue culture plastic dish at 0.15 × 10⁴ cells/well for A549 cells and SUIT-2 cells, and at 0.45 × 10⁴ cells/well for SW1990 cells. 1.1 pmol of siRNA was added into 5 µL of Opti-MEM I Reduced Serum Medium and gently mixed. Next, 0.12 µL of Lipofectamine RNAiMAX was diluted into 4.88 µL of Opti-MEM I Reduced Serum Medium and gently mixed. The siRNA dilution and the Lipofectamine RNAiMAX dilution were combined, gently mixed, and then incubated at room temperature for 15 minutes. During this period, the medium was replaced with 100 µL of Opti-MEM I Reduced Serum Medium. After the incubation for 15 minutes, the complex of siRNA and Lipofectamine RNAiMAX was added to the cells and incubated at 37°C in the atmosphere containing 5% CO₂.

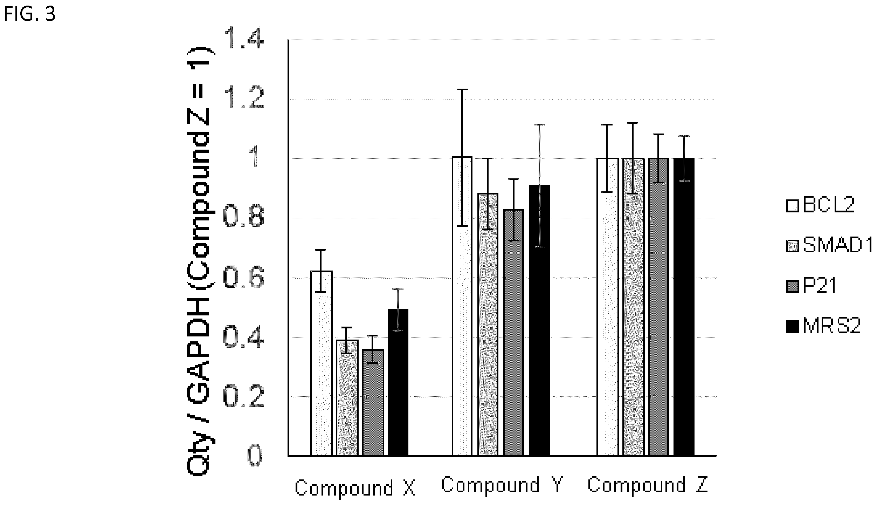

After the incubation for 5 hours, the medium was replaced with 3 mL of each medium containing 10% FBS. On day 4 after the transfection, Hoechst 33342 (Thermo Fisher Scientific Inc., #H3570) and propidium iodide (Wako Pure Chemical Industries, Ltd., #169-26281) were added at final concentrations of 5 µg/mL and 2 µg/mL, respectively, to the medium. Live and dead cell numbers were counted using Celigo^{®} Image Cytometer (Nexcelom Bioscience, LLC, #Celigo-106-0448) in which the number of cells stained with Hoechst 33342 was regarded as the total of the live and dead cell numbers and the number of cells stained with propidium iodide was regarded as the dead cell number. As shown in results in Figures 2-1 to 2-3, Compound X suppressed the proliferation of all the cancer cells more strongly than Compound Y did, and the ratio of cell death was also larger for Compound X than for Compound Y.

### Example 3: Influence of Compound X on gene expression other than gene expression of BcL-XL

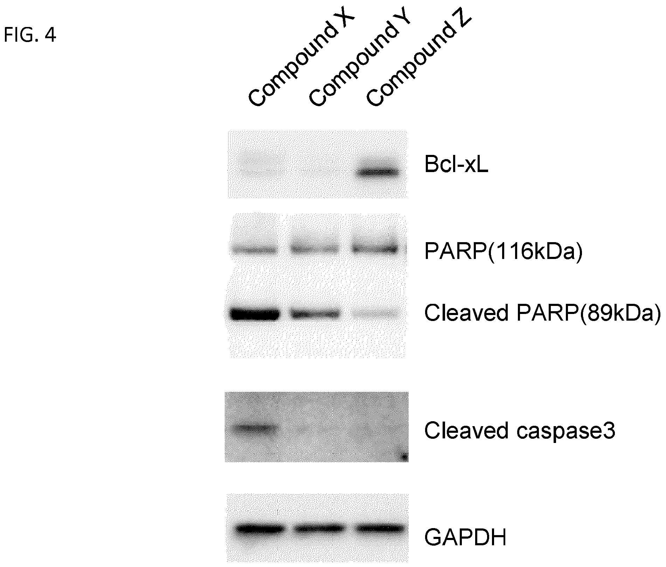

In the same way as in Example 1, siRNA was introduced into HCT116 cells and incubated, and RNA was then recovered and reversely transcribed into cDNA. The mRNA levels of BCL2, SMAD1, P21, and MRS2 were quantified by quantitative PCR using the obtained cDNA and 7300 Real Time PCR System (Applied BioSystems, Inc.). As shown in results in Figure 4, Compound Y did not suppress the expression of BCL2, SMAD1, P21, or MRS2, whereas Compound X suppressed the expression of all of these genes.

### Example 4: Testing of apoptosis-inducing activity of Compound X

SiRNA was introduced into HCT116 cells and incubated in the same way as in Example 1 except that the density of the seeded cells was 0.2 × 10⁵ cells/well. On day 3 after the transfection, cell extracts were prepared, and change in the expression of apoptosis signals activated caspase-3 and activated PARP was analyzed by Western blot.

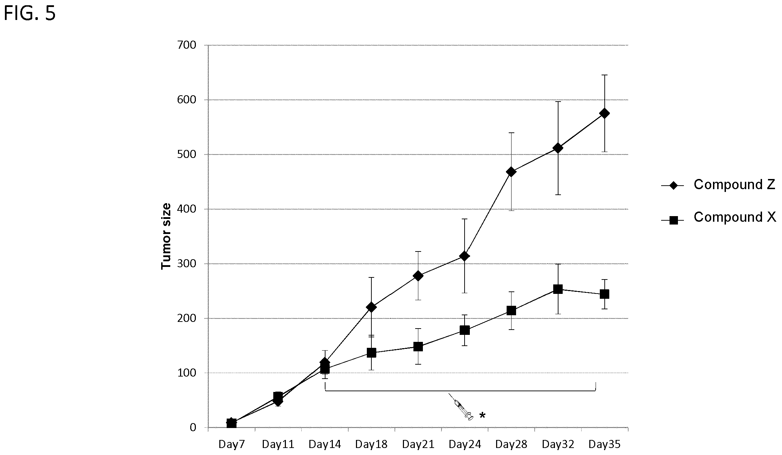

Western blot was performed as follows: the cells were washed with ice-cooled PBS and then lysed by the addition of TNE lysis buffer (1% NP-40, 50 mM Tris-HCl, 150 mM NaCl, 1 mM EDTA, complete Mini EDTA-free (F. Hoffmann-La Roche, Ltd.), and PhosSTOP (F. Hoffmann-La Roche, Ltd.), pH 7.5) and incubation for 30 minutes under ice cooling. Then, the cells were centrifuged for 15 minutes under conditions of 15000 rpm and 4°C, and the supernatant was used as cell extracts. Proteins in the obtained cell extracts were quantified using Micro BCA Protein Assay Kit (Thermo Fisher Scientific Inc.). Red Loading Buffer Pack (New England Biolabs Inc.) was added to 10 µg of the cell extracts, which were then heat-treated (100°C, 5 min) for denaturation. The proteins were separated by SDS-PAGE using SuperSep^{™} Ace (Wako Pure Chemical Industries, Ltd.). The proteins thus separated were transferred to PVDF transfer membrane (Immobilon-P; Merck Millipore) using a semidry blotting apparatus (Bio-Rad Laboratories, Inc.). The membrane was blocked by incubation at room temperature for 1 hour in PBS supplemented with 5% skimmed milk/0.05% Tween 20 (hereinafter, abbreviated to PBS-T). Subsequently, the membrane was incubated at 4°C for 16 hours with various primary antibodies (Bcl-xL (54H6) Rabbit mAb #2764 (Cell Signaling Technology, Inc. (CST)), PARP Antibody #9542 (CST), Cleaved Caspase-3 (Asp175) (5A1E) Rabbit mAb #9664 (CST), and Anti-GAPDH antibody [6C5] (Abcam plc)) diluted with PBS-T. The membrane was washed with PBS-T and then incubated at room temperature for 60 minutes with corresponding HRP-conjugated anti-mouse or anti-rabbit IgG (CST). The membrane was washed with PBS-T and then reacted with SuperSignal^{™} West Femto Maximum Sensitivity Substrate (Thermo Fisher Scientific Inc.), followed by chemiluminescence detection using chemidoc (Bio-Rad Laboratories, Inc.). In the washing between the operations, shaking for 5 minutes was performed three times using PBS-T. As shown in results in Figure 5, the apoptosis signals activated caspase-3 and activated PARP were observed by Compound X, indicating that apoptosis was induced. On the other hand, no activated caspase-3 and only a slight level of activated PARP were observed for Compound Y.

### Example 5: Testing of antitumor effect of Compound X in vivo

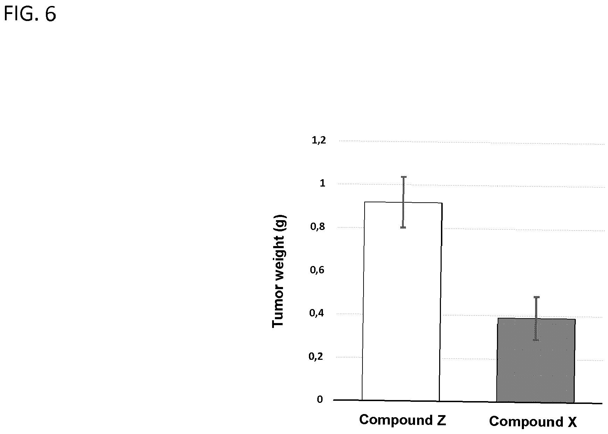

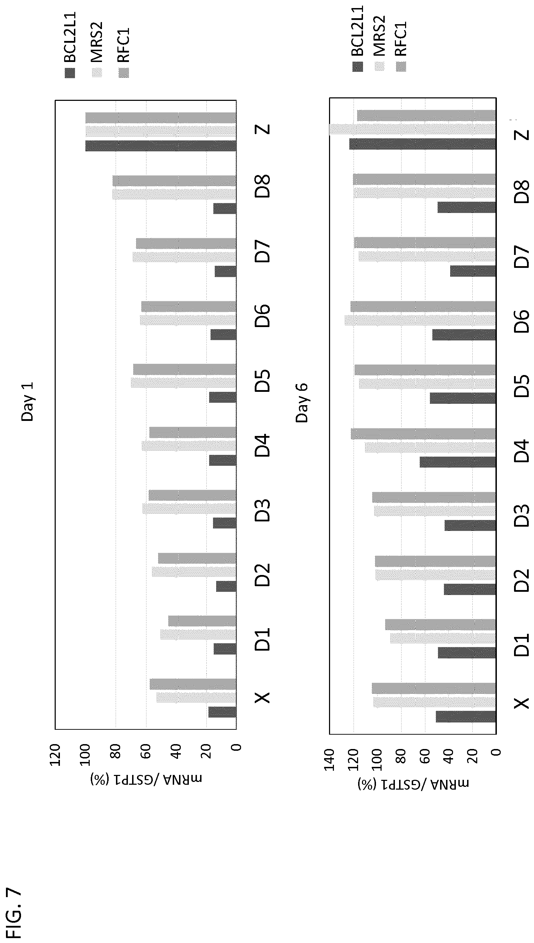

1.0 × 10⁵ colon cancer cell line HCT116 cells were subcutaneously inoculated to each BALB/c nu/nu mouse (6 to 8 weeks old, female, n = 4, purchased from CLEA Japan, Inc.) to prepare a cancer-bearing mouse. From day 1 after the inoculation, Compound X or Compound Z was intratumorally administered twice a week at a dose of 1 mg per g of mouse body weight, and the volume of tumor was measured with a caliper. The delivery of each compound employed LipoTrust^{™} *EX* Oligo *<in vivo>* (Hokkaido System Science Co., Ltd.). On day 35 after the inoculation, the mouse was euthanized, and its tumor weight was measured. Change in tumor volume is shown in Figure 6, and the comparison of the tumor weight is shown in Figure 7. As is evident from both the diagrams, Compound X also markedly suppressed the growth of tumor *in vivo.*

### Example 6: Potentiation of ability to suppress cancer cell proliferation by changing nucleotide of CUGACUC sequence to DNA

The ability to suppress cell proliferation was evaluated by the action of siNA in which a nucleotide of the CUGACUC sequence was changed to DNA.

The siNA used was Compound X as well as siNAs consisting of the following nucleotide sequences.
Compound D1 (Comprising SEQ ID NO: 2 in antisense strand)
Sense strand: 5' -GGAUACAGCUGGAGUCAGUtt-3' (SEQ ID NO: 38)
Antisense strand: 5' -AcUGACUCCAGCUGUAUCCtt-3' (SEQ ID NO: 42)
Compound D2 (Comprising SEQ ID NO: 3 in antisense strand)
Sense strand: 5' -GGAUACAGCUGGAGUCAGUtt-3' (SEQ ID NO: 38)
Antisense strand: 5' -ACtGACUCCAGCUGUAUCCtt-3' (SEQ ID NO: 43)
Compound D3 (Comprising SEQ ID NO: 4 in antisense strand)
Sense strand: 5' -GGAUACAGCUGGAGUCAGUtt-3' (SEQ ID NO: 38)
Antisense strand: 5' -ACUgACUCCAGCUGUAUCCtt-3' (SEQ ID NO: 44)
Compound D4 (Comprising SEQ ID NO: 5 in antisense strand)
Sense strand: 5' -GGAUACAGCUGGAGUCAGUtt-3' (SEQ ID NO: 38)
Antisense strand: 5' -ACUGaCUCCAGCUGUAUCCtt-3' (SEQ ID NO: 45)
Compound D5 (Comprising SEQ ID NO: 6 in antisense strand)
Sense strand: 5' -GGAUACAGCUGGAGUCAGUtt-3' (SEQ ID NO: 38)
Antisense strand: 5' -ACUGAcUCCAGCUGUAUCCtt-3' (SEQ ID NO: 46)
Compound D6 (Comprising SEQ ID NO: 7 in antisense strand)
Sense strand: 5' -GGAUACAGCUGGAGUCAGUtt-3' (SEQ ID NO: 38)
Antisense strand: 5' -ACUGACtCCAGCUGUAUCCtt-3' (SEQ ID NO: 47)
Compound D7 (Comprising SEQ ID NO: 8 in antisense strand)
Sense strand: 5' -GGAUACAGCUGGAGUCAGUtt-3' (SEQ ID NO: 38)
Antisense strand: 5' -ACUGACUcCAGCUGUAUCCtt-3' (SEQ ID NO: 48)
Compound D8 (Comprising CUgAcUc sequence in antisense strand)
Sense strand: 5' -GGAUACAGCUGGAGUCAGUtt-3' (SEQ ID NO: 38)
Antisense strand: 5' -ACUgAcUcCAGCUGUAUCCtt-3' (SEQ ID NO: 49)

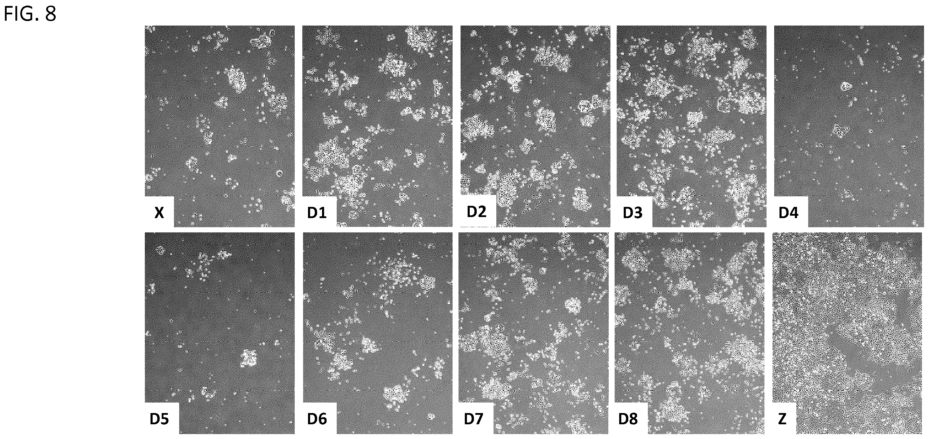

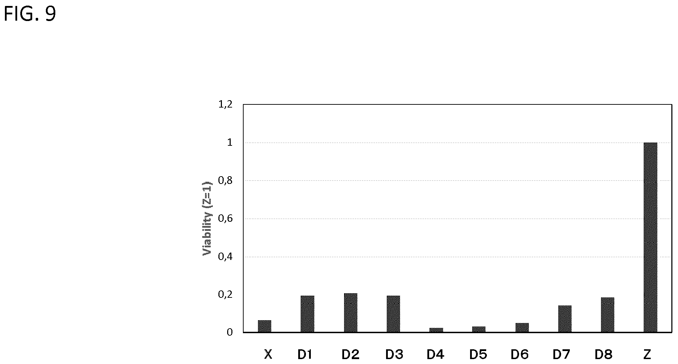

Lung cancer cell line A549 was cultured in DMEM medium containing 10% inactivated fetal bovine serum (FBS), and 100 U/mL penicillin and 100 µg/mL streptomycin as antibiotics (10% FBS/DMEM) under conditions of 37°C and 5% CO_{2.} In each well of a 6-well plate, 500 µL of Opti-MEM I Reduced Serum Medium containing 50 pmol of siNA and 500 µL of Opti-MEM containing 15 µL of Lipofectamine RNAiMAX were mixed and left at room temperature for 15 minutes. 2 mL of A549 was added thereto at 0.25 × 10⁵ cells/mL and cultured at 37°C. 1 day later or 6 days later, light field images were taken under an inverted microscope. Then, RNA was recovered and reversely transcribed into cDNA. Then, the mRNA levels of BcL-XL, MRS2 and RFC1 were quantified by quantitative PCR using 7300 Real Time PCR System (Applied BioSystems, Inc.). Results in Figure 7 are indicated by a relative value of the obtained numerical value normalized with the expression level of GSTP1 with a value for a control (Compound Z) defined as 100%. Cell viability was evaluated on the basis of the amplification level of GSTP1 having stable expression. The results are shown in Figures 8 and 9. All the siNAs in which a nucleotide of the CUGACUC sequence was changed to DNA exhibited a reduction in cell viability. The siNA in which the nucleotide at position 5, 6 or 7 from the 5' end of the antisense strand was changed to DNA had a particularly high effect of suppressing cell proliferation.

Those skilled in the art will understand that many various modifications can be made in the present invention without departing from the spirit of the present invention. Thus, it should be understood that the modes of the present invention described in the present specification are given merely for illustrative purposes and are not intended to limit the scope of the present invention.

## Claims

1. An RNAi molecule comprising the nucleotide sequence of SEQ ID NO: 1 in an antisense strand.

2. The RNAi molecule according to claim 1, wherein the nucleotide sequence of SEQ ID NO: 1 is located at positions 2 to 8 from 5' of the antisense strand.

3. The RNAi molecule according to claim 1 or 2, which suppresses the protein expression of a member of the BcL2 family.

4. The RNAi molecule according to claim 3, wherein the member of the BcL2 family is BcL-XL.

5. The RNAi molecule according to any one of claims 1 to 4, wherein the antisense strand comprises the nucleotide sequence of SEQ ID NO: 21.

6. The RNAi molecule according to any one of claims 1 to 5, wherein the nucleotide sequence of SEQ ID NO: 1 is selected from the nucleotide sequence of SEQ ID NOs: 5 to 7

7. A pharmaceutical composition comprising the RNAi molecule according to any one of claims 1 to 6, and optionally a pharmaceutically acceptable additive.

8. The RNAi molecule according to any one of claims 1 to 6 or the composition according to claim 7 for use in a treatment of a cancer.

9. The RNAi molecule according to claim 8, wherein the cancer expresses BcL-XL.

10. The RNAi molecule or the pharmaceutical composition according to claim 9, wherein the cancer is selected from the group consisting of brain tumor, head and neck cancer, breast cancer, lung cancer, oral cancer, esophageal cancer, stomach cancer, duodenal cancer, appendix cancer, colon cancer, rectal cancer, liver cancer, pancreatic cancer, gallbladder cancer, bile duct cancer, anal cancer, kidney cancer, ureteral cancer, bladder cancer, prostate cancer, penile cancer, testicular cancer, uterine cancer, cervical cancer, ovarian cancer, vulvar cancer, vaginal cancer, skin cancer, fibrosarcoma, malignant fibrous histiocytoma, liposarcoma, rhabdomyosarcoma, leiomyosarcoma, angiosarcoma, Kaposi's sarcoma, lymphangiosarcoma, synovial sarcoma, chondrosarcoma, osteosarcoma, myeloma, lymphoma and leukemia.

11. A method for treating a cancer, comprising administering an effective amount of the RNAi molecule according to any one of claims 1 to 6 and 8 to 10 or the composition according to any one of claims 7to 10 to a subject in need thereof.
